# NOUVEAU FASCICULE DE BREVET EUROPEEN

(11) **EP 3 174 994 B2**
(45) Date de publication et mention de la décision concernant l'opposition: **07.12.2022**
(45) Mention de la délivrance du brevet: 30.01.2019
(21) Numéro de dépôt: 15756680.3
(22) Date de dépôt: 29.07.2015
(51) Int. Cl.: C12Q 1/18, G01N 33/68, C12Q 1/04

(54) **CARACTERISATION DE MICRO-ORGANISMES PAR MALDI-TOF**
CHARAKTERISIERUNG VON MIKROORGANISMEN ÜBER MALDI-TOF
CHARACTERIZATION OF MICROORGANISMS VIA MALDI-TOF

(30) Priorité: 30.07.2014 FR 1457389
(43) Date de publication de la demande: 07.06.2017
(60) Demande divisionnaire: 19152443.8
(73) Titulaire: Biomérieux, 69280 Marcy-L'Etoile (FR)
(72) Inventeur: RAMJEET, Mahendrasingh, F-69006 Lyon (FR); SURRE, Jérémy, F-01120 Montluel (FR); FERULLO, Audrey, F-13003 Marseille (FR)
(74) Mandataire: Cabinet Beau de Loménie
(86) Numéro de dépôt international: PCT/FR2015/052105
(87) Numéro de publication internationale: WO 2016/016580

(56) Documents cités:
- WO-A1-2012/023845
- WO-A1-2012/113699
- WO-A2-2005/001475
- DE-A1- 10 140 499
- GB-A- 2 438 066
- VAN BAAR B L M: "Characterisation of bacteria by matrix-assisted laser desorption/ionisation and electrospray mass spectrometry", FEMS MICROBIOLOGY REVIEWS, ELSEVIER, AMSTERDAM; NL, vol. 24, 1 janvier 2000 (2000-01-01), pages 195-219, XP002980425, ISSN: 0168-6445, DOI: 10.1016/S0168-6445(99)00036-4
- ANJA FREIWALD ET AL: "Phylogenetic classification and identification of bacteria by mass spectrometry", NATURE PROTOCOLS, vol. 4, no. 5, 1 mai 2009 (2009-05-01), pages 732-742, XP055182663, ISSN: 1754-2189, DOI: 10.1038/nprot.2009.37
- N. VALENTINE ET AL: "Effect of Culture Conditions on Microorganism Identification by Matrix-Assisted Laser Desorption Ionization Mass Spectrometry", APPLIED AND ENVIRONMENTAL MICROBIOLOGY, vol. 71, no. 1, 1 janvier 2005 (2005-01-01), pages 58-64, XP055182668, ISSN: 0099-2240, DOI: 10.1128/AEM.71.1.58-64.2005
- ZHOUXIN SHEN ET AL: "A Mass Spectrometry Plate Reader: Monitoring Enzyme Activity and Inhibition with a Desorption/Ionization on Silicon (DIOS) Platform", CHEMBIOCHEM, vol. 5, no. 7, 1 juillet 2004 (2004-07-01) , pages 921-927, XP055182670, ISSN: 1439-4227, DOI: 10.1002/cbic.200400008
- A. VERROKEN ET AL: "Evaluation of Matrix-Assisted Laser Desorption Ionization-Time of Flight Mass Spectrometry for Identification of Nocardia Species", JOURNAL OF CLINICAL MICROBIOLOGY, vol. 48, no. 11, 22 septembre 2010 (2010-09-22), pages 4015-4021, XP055183664, ISSN: 0095-1137, DOI: 10.1128/JCM.01234-10
- SARANGI N.P. ATHUKORALA et al.: "Identification of antifungal antibiotics of Bacillus species isolated from different microhabitats using polymerase chain reaction and MALDI-TOF mass spectrometry", Can. J. Microbiol., vol. 55, 21 August 2009 (2009-08-21), pages 1021-1032,
- Johansson et al: BMC Microbiology, 2014, 44:89 (10.04.2014)
- Ray Lidgard et al: Rapid Communications in Mass Spectrometry. Vol. 9, 128-132 (1995)

## Description

La présente invention concerne le domaine de la microbiologie. Plus précisément, l'invention concerne la caractérisation d'une population d'au moins un micro-organisme en utilisant la spectrométrie de masse, et en particulier, la spectrométrie de masse (SM) par désorption-ionisation assistée par matrice et mesure du temps de vol dite MALDI-TOF.

La technique MALDI-TOF est utilisée depuis quelques années pour réaliser une identification rapide de micro-organismes, au niveau de l'espèce. Différents appareillages adaptés à une telle caractérisation sont commercialisés par la demanderesse, et par les sociétés Bruker Daltonics et Andromas notamment.

L'identification d'un micro-organisme est réalisée à partir du spectre de masse MALDI-TOF des protéines les plus abondantes dans le micro-organisme, par comparaison avec des données de référence permettant l'identification de la famille, du genre et le plus souvent de l'espèce du micro-organisme. En routine, le protocole mis en oeuvre comprend le dépôt d'au moins une portion de colonie du micro-organisme sur une plaque MALDI, l'ajout d'une matrice adaptée à la technique MALDI, l'acquisition du spectre de masse et l'identification de l'espèce par comparaison avec des données de référence stockées dans une base de données.

Plus récemment, la technique MALDI a également été utilisée pour permettre de détecter la résistance d'un micro-organisme à un antibiotique, et notamment pour identifier un phénotype, responsable de l'hydrolyse des antibiotiques du type bêta-lactamine, du fait de la sécrétion d'enzymes de type bêta-lactamase, et notamment, de type carbapénémase. A cet égard, on peut citer les demandes US 2012/0196309 et WO 2012/023845. La caractérisation de la résistance par MALDI-TOF implique la détection de la forme hydrolysée et/ou la perte de la forme native de l'antibiotique bêta-lactamine après incubation avec ledit antibiotique de l'échantillon susceptible de contenir un micro-organisme capable de produire une bêta-lactamase.

Bien que dans ces demandes de brevet, la préparation de l'échantillon à déposer ne soit pas décrite de manière détaillée, il est, en général, envisagé de mélanger préalablement le micro-organisme avec l'agent antibiotique, puis de réaliser le dépôt du mélange sur la plaque MALDI ou d'utiliser un lysat de micro-organisme(s) (voir WO 2012/023845, revendication 15 notamment).

Le document WO 2012/113699 envisage, quant à lui, d'utiliser la spectrométrie de masse pour évaluer l'effet inhibiteur d'une substance sur des bêta-lactamases, cette évaluation pouvant se faire en présence de bactéries.

Les publications suivantes (Hrabak, Walkova et al. 2011, Hooff, van Kampen et al. 2012, Hrabak, Studentova et al. 2012, Sparbier, Schubert et al. 2012) donnent une description plus détaillée des protocoles de préparation d'échantillons à mettre en oeuvre, qui comportent les étapes suivantes :
- préparation d'un inoculum comprenant des colonies du micro-organisme,
- centrifugation de l'inoculum,
- resuspension du culot bactérien avec une solution de l'antibiotique avec ou sans réactif de lyse,
- incubation pendant une durée de 30 minutes à 3 heures,
- centrifugation,
- transfert d'un microlitre du surnageant sur une plaque MALDI et ajout d'un microlitre de matrice, analyse par spectrométrie de masse MALDI-TOF.

La préparation de l'échantillon préalable à une détection de résistance par la technique MALDI-TOF est donc longue et fastidieuse. En effet, la procédure utilisée pour la détection de résistance par la technique MALDI-TOF nécessite la préparation d'un inoculum de concentration élevée et/ou une ou plusieurs étapes de centrifugation. Ces étapes nécessitent des matériaux, des consommables, un équipement spécifique et sont donc consommatrices à la fois en consommables, en temps et en travail d'opérateur. Aussi, cette préparation préalable rend difficile l'utilisation en routine de la technique MALDI-TOF pour la détection de résistance, étant donné qu'il n'est pas envisageable de réaliser la caractérisation d'un grand nombre d'échantillons par jour. En effet, il y a bien souvent un manque d'adéquation entre la disponibilité de l'appareil pour réaliser l'identification du phénomène de résistance par la technique MALDI-TOF, et la disponibilité des échantillons obtenus après l'étape obligatoire de préparation. Un autre problème réside dans le fait que les bactéries sont incubées avec l'antibiotique, dans des volumes de l'ordre de 10 à 50 µl, ce qui présente un risque potentiel important de réduire les interactions enzyme-antibiotique. Par conséquent, une diminution de l'efficacité de l'activité enzymatique peut être rencontrée, ce qui affecte la sensibilité de détection et donc, la robustesse de la technique, en particulier, dans le cas de micro-organismes connus pour produire ou sécréter des faibles quantités d'enzymes.

La détection de résistance à un agent antimicrobien a également été décrite dans les demandes WO 2011/045544 et US 2012/0196309 comme pouvant être réalisée par d'autres techniques de spectrométrie de masse, comme la MS-MS et la MRM. Dans ce cas, l'analyse par spectrométrie de masse et donc l'ionisation ne sont pas réalisées sur le micro-organisme, mais sur les protéines obtenues après diverses opérations de purification.

Dans le cadre de l'invention, les inventeurs proposent de mettre en oeuvre une nouvelle méthode de préparation d'une zone de caractérisation d'un micro-organisme par technique MALDI, permettant l'identification d'une résistance à un antibiotique, qui soit simple à mettre en oeuvre. Par ailleurs, cette nouvelle méthode est compatible avec l'obtention de différentes caractérisations de l'échantillon présent sur la zone de caractérisation, dont une au moins correspond à l'identification d'une résistance à un antibiotique et une autre pouvant correspondre à l'identification d'un micro-organisme.

L'invention a également pour objectif de proposer un procédé de caractérisation d'un échantillon contenant au moins un micro-organisme par la technique MALDI-TOF, qui permette de savoir si une population d'un micro-organisme présent est ou non résistante à au moins un agent antimicrobien, dans un temps relativement court, de l'ordre de quelques heures à une heure, voire moins.

Aussi, l'invention utilise un procédé de préparation d'une zone de caractérisation d'une plaque d'analyse pour la réalisation d'au moins une caractérisation d'une population d'au moins un micro-organisme en présence d'au moins un agent antimicrobien, ladite caractérisation mettant en oeuvre une analyse par spectrométrie de masse au cours de laquelle la population d'au moins un micro-organisme déposée sur la zone d'analyse est soumise à au moins une étape d'ionisation par bombardement de la zone d'analyse par un faisceau laser selon la technique de spectrométrie de masse par désorption-ionisation assistée par matrice nommée MALDI,
caractérisé en ce que le procédé de préparation de la zone d'analyse comprend les étapes successives suivantes :
- une étape consistant à disposer d'une plaque d'analyse pour une caractérisation par la technique MALDI, comprenant au moins une zone d'analyse porteuse d'un agent antimicrobien,
- une étape de dépose de la population d'au moins un micro-organisme sur ladite zone d'analyse au contact de l'agent antimicrobien,
- une étape d'incubation, consistant à conserver la plaque d'analyse, dans des conditions et pendant un temps suffisant, pour permettre l'interaction de l'agent antimicrobien et du micro-organisme présent,
- une étape de dépose sur la zone d'analyse, d'une matrice adaptée à la technique MALDI.

De manière préférée, dans le cadre du procédé de préparation utilisé dans l'invention, une population d'un seul micro-organisme à caractériser est déposée. Ainsi, la zone de caractérisation peut ensuite être utilisée pour la caractérisation d'un seul micro-organisme.

Selon l'invention, la population de micro-organisme(s) déposée est préparée, sans étape de mise en contact préalable avec un agent antimicrobien, c'est-à-dire que la population de micro-organisme(s) déposée ne se trouve pas avant son dépôt en mélange avec l'agent antimicrobien présent sur la zone de caractérisation.

Le procédé selon l'invention sera avantageusement mis en oeuvre sur une population de micro-organisme(s) obtenue après une étape de concentration, enrichissement et/ou purification et/ou qui correspond à une colonie ou à une fraction de colonie obtenue après croissance sur un milieu adapté, notamment un milieu gélosé.

Dans le cadre de l'invention, l'agent antimicrobien est, par exemple, sélectionné de manière à permettre l'identification de la résistance due à la production de bêta-lactamase, et notamment de carbapénémase.

L'agent antimicrobien est, le plus souvent, un antibiotique, de préférence sélectionné parmi les pénicillines, les céphalosporines, les céphamycines, les carbapénèmes, les monobactames et en particulier parmi l'ampicilline, l'amoxicilline, la ticarcilline, la piperacilline, la céfalotine, le céfuroxime, la céfoxitine, le céfixime, le céfotaxime, la céftazidime, la ceftriaxone, la cefpodoxime, le céfépime, l'aztreonam, l'ertapénème, l'imipénème, le méropénème et le faropénème.

Le procédé de préparation utilisé dans l'invention peut comprendre une étape de fonctionnalisation permettant d'obtenir la plaque d'analyse pour une caractérisation par la technique MALDI, comprenant au moins une zone d'analyse porteuse d'un agent antimicrobien, dite fonctionnalisée, ladite étape de fonctionnalisation étant, de préférence, réalisée par dépôt d'une solution aqueuse de l'agent antimicrobien, suivi d'un séchage.

L'invention a également pour objet un procédé de caractérisation d'une population d'au moins un micro-organisme tel que défini dans la revendication 1.

En particulier, l'analyse par spectrométrie de masse selon la technique MALDI-TOF, permettant de conclure si une population d'un micro-organisme résistant à l'agent antimicrobien est présente sur la zone de caractérisation, consiste à vérifier la présence de l'agent antimicrobien et/ou d'un produit de dégradation de l'agent antimicrobien.

Le procédé de caractérisation selon l'invention comprend au moins deux caractérisations. La caractérisation comprend, en plus d'une analyse par spectrométrie de masse par désorption-ionisation assistée par matrice et mesure du temps de vol dite MALDI-TOF, d'une population d'un micro-organisme déposée sur la zone de caractérisation, permettant de conclure si une population d'un micro-organisme résistant à l'agent antimicrobien est présente sur la zone de caractérisation, l'identification de la famille, du genre, ou, de préférence, de l'espèce d'une population d'un micro-organisme déposée sur la zone de caractérisation.

Le procédé de caractérisation selon l'invention comprend :
- l'identification de la famille, du genre, ou, de préférence, de l'espèce d'une population d'un micro-organisme déposée sur la zone de caractérisation, par mise en oeuvre d'une première analyse par spectrométrie de masse de type MALDI correspondant à une première étape d'ionisation d'une zone de caractérisation, et utilisant pour l'analyse une première calibration, et
- la détermination de la présence éventuelle sur la zone de caractérisation d'une population d'un micro-organisme résistant à l'agent antimicrobien, par mise en oeuvre d'une deuxième analyse par spectrométrie de masse selon la technique MALDI-TOF correspondant à une seconde étape d'ionisation de la même zone de caractérisation, et utilisant pour l'analyse une deuxième calibration.

L'identification de la famille, du genre, ou, de préférence, de l'espèce d'une population d'un micro-organisme, et la détermination de la résistance éventuelle à l'agent antimicrobien concerne, de préférence, le même micro-organisme.

Le procédé de caractérisation d'une population d'au moins un micro-organisme, selon l'invention est caractérisé en ce que :
- la première et la seconde analyse sont mises en oeuvre, respectivement, par une première étape et une seconde étape d'ionisation distinctes d'une même zone de caractérisation comprenant la population d'au moins un micro-organisme et l'agent antimicrobien,
- la première analyse utilise une première calibration, et la seconde analyse utilise une seconde calibration différente de la première calibration.

De préférence, la population déposée comprend un seul micro-organisme à caractériser.

Un tel procédé de caractérisation met en oeuvre un procédé de préparation d'une zone de caractérisation d'une plaque d'analyse.

Dans le cadre de l'invention, la même zone de caractérisation et donc la même population de micro-organisme(s) est soumise successivement à deux ionisations permettant d'obtenir les deux caractérisations souhaitées : la caractérisation d'un phénomène de résistance d'un micro-organisme à un agent antimicrobien et l'identification du micro-organisme. L'invention a également pour objet un procédé de fonctionnalisation d'une zone d'analyse d'une plaque d'analyse adaptée à la technique MALDI, caractérisé en ce que le procédé de fonctionnalisation de la zone d'analyse comprend :
- une étape de fonctionnalisation de la zone d'analyse rendant la zone d'analyse porteuse d'un agent antimicrobien.

L'étape de fonctionnalisation peut être réalisée par dépôt d'une solution aqueuse de l'agent antimicrobien, suivi d'un séchage.

Un autre objet de la description concerne une plaque d'analyse destinée à recevoir une population d'au moins un micro-organisme à caractériser sur une zone d'analyse par spectrométrie de masse selon la technique MALDI, caractérisée en ce qu'elle comporte un agent antimicrobien déposé, en particulier sous la forme d'un dépôt solide, sur ladite zone d'analyse de la plaque d'analyse, formant une zone d'analyse dite fonctionnalisée. En particulier, dans une telle plaque d'analyse, la ou les zone(s) d'analyse fonctionnalisée(s), porteuse(s) de l'agent antimicrobien, ne comporte(nt) pas de micro-organisme en une quantité détectable par la technique MALDI et/ou la ou les zone(s) d'analyse fonctionnalisée(s), porteuse(s) de l'agent antimicrobien, est(sont) sèche(s).

Par « sèche », on entend en particulier que l'agent antimicrobien ne se trouve pas en solution, mais sous la forme d'un dépôt solide qui adhère à la zone d'analyse sur laquelle il est déposé, En particulier, le maintien d'un agent antimicrobien sur une zone d'analyse dite fonctionnalisée pourra être assuré par liaison électrostatique, ionique, covalente, d'affinité ou encore grâce à un agent adhésif. L'agent adhésif sera, notamment, un polymère soluble dans l'eau. A titre d'exemple d'agent adhésif pouvant être utilisé pour l'immobilisation de l'agent anti-microbien sur la ou les zones d'analyse, on peut citer l'heptakis(2,6-di-*O*-méthyl)-β-cyclodextrine.

Selon un mode de réalisation particulier, dans une telle plaque d'analyse destinée à recevoir une population d'au moins un micro-organisme à caractériser, la ou les zone(s) d'analyse fonctionnalisée(s), sera(ont) chacune porteuse(s) d'un seul agent antimicrobien,

La plaque d'analyse destinée à recevoir une population d'au moins un micro-organisme décrite dans la description pourra être conditionnée dans un emballage hermétique. L'emballage hermétique sera, notamment, adapté pour protéger la plaque de la lumière et de l'humidité.

La ou les zone(s) d'analyse(s) fonctionnalisée(s) pourront être obtenue(s) par dépôt d'une solution aqueuse de l'agent antimicrobien, suivi d'un séchage.

De telles plaques pourront comporter plusieurs zones d'analyse fonctionnalisées, chacune étant porteuse d'un agent antimicrobien, avec notamment au moins une première zone d'analyse fonctionnalisée porteuse d'un premier agent antimicrobien, et une seconde zone d'analyse fonctionnalisée, distincte de la première zone, porteuse d'un second agent antimicrobien distinct du premier agent antimicrobien.

Le plus souvent, de telles plaques comportent au moins une zone d'analyse de référence, destinée à recevoir ultérieurement une population d'un micro-organisme de référence, et la surface de la zone d'analyse de référence est dépourvue d'agent antimicrobien.

L'invention concerne également l'utilisation d'une plaque d'analyse décrite dans la description, dans un procédé de caractérisation selon l'invention.

La description ci-après, en référence aux figures annexées permet de mieux comprendre l'invention.

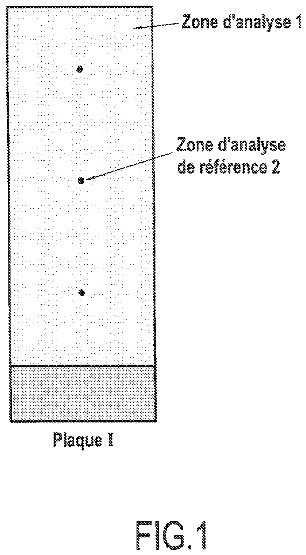

La **Figure 1** est une vue schématique de dessus d'une plaque MALDI.

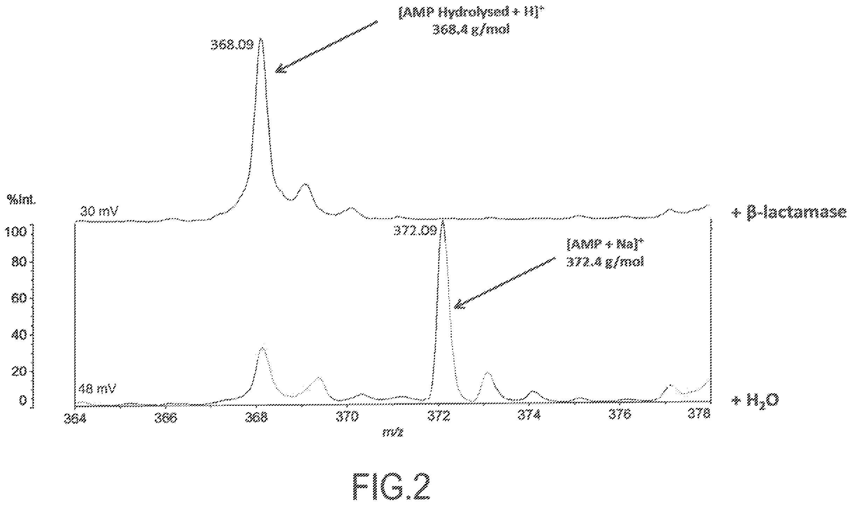

Les **Figures 2** à **4** sont des spectres de masse obtenus dans les exemples par la technique MALDI-TOF. Sur ces figures, l'échelle d'intensité est une échelle relative par rapport au pic le plus intense du spectre. Par exemple, sur une gamme de masse choisie (notamment de 200 à 1200 m/z), si le pic le plus intense est de 100 mV, il sera noté 100% (comme mentionné à gauche des spectres). Les pics moins intenses seront notés par rapport au pic le plus intense : un pic d'intensité 75 mV atteindra donc 75% de l'échelle (sur le spectre contenant le pic d'intensité maximale de 100 mV). Par conséquent, sur un même spectre, l'intensité des pics entre les souches ne peut être comparée. Par contre, ces spectres permettent de comparer, pour une même souche, le niveau d'intensité entre le pic natif de l'agent antimicrobien et celui de son produit de dégradation. Il sera également possible de comparer, pour une même souche, le niveau d'intensité entre les pics natifs ou hydrolysés et un pic contrôle n'étant pas soumis aux variations induites par l'activité biologique/enzymatique du microorganisme à tester. Pourront être considérés comme pics contrôles, un pic de la matrice HCCA, le pic d'un peptide ou une molécule de référence ajouté(e) à la matrice ou préalablement séché(e) sur la zone d'analyse.

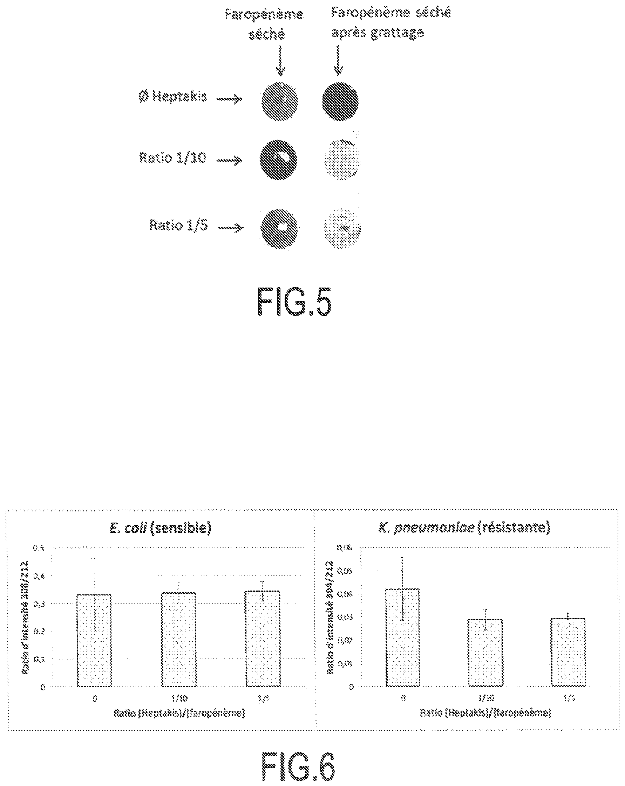

**La** **Figure 5** montre l'aspect de zones d'analyse (spots) sur lesquelles un antibiotique, le faropénème a été déposé, en présence et en absence d'agent adhésif (l'Heptakis), avant et après grattage avec une oese.

La **Figure 6** montre la variation des ratios d'intensités des pics du faropénème natif et du faropénème hydrolysé, obtenus par la technique MALDI-TOF par rapport au pic contrôle de l'HCCA en fonction des ratios [Heptakis]/[faropénème].

La **Figure 7** présente les spectres de masse obtenus lors de la deuxième série d'acquisitions de l'Exemple 5.

La **Figure 8** montre la variation des ratios d'intensités 304/308 et 304/330 en fonction de la concentration en inoculum utilisée pour les 2 souches testées à l'Exemple 5.

La **Figure 9** représente un modèle de boitier intégrant une plaque MALDI qui pourrait être adapté au dépôt d'une population de micro-organisme sous forme liquide.

### Plaques d'analyse MALDI

Une plaque d'analyse MALDI présente, au moins une et, en général plusieurs zones d'analyse. Les zones d'analyse forment un spot, le plus souvent de forme circulaire. Afin de favoriser l'ionisation ultérieure, au moins au niveau de la ou des zones d'analyse, la surface de la plaque est conductrice. A titre d'exemple, une telle plaque d'analyse est formée d'un polymère tel que le polypropylène, ledit polymère étant recouvert d'une couche d'acier inoxydable. Le polymère peut contenir un matériel conducteur tel que le noir de charbon. Une telle plaque peut-être, par exemple, celle commercialisée par la société Shimadzu, sous la référence Fleximass^{™} DS disposable MALDI targets.

Différentes plaques MALDI sont disponibles dans le commerce telles que les plaques Fleximass DS de bioMérieux (jetables ou réutilisables) et les plaques Maldi Biotarget de Bruker Daltonics. De telles plaques **I** comprennent le plus souvent de 48 à 96 zones d'analyse **1** ou spots, et au moins une, voire deux ou trois zones d'analyse de référence **2,** qui peuvent être comme dans l'exemple illustré sur la **Figure 1** de taille différente des zones d'analyse,

Dans le cadre de l'invention, on nomme « zone de caractérisation » une zone d'analyse porteuse d'un agent antimicrobien, d'une population de micro-organisme(s) et d'une matrice adaptée à la technique MALDI.

### Fonctionnalisation de la zone d'analyse

Par « agent antimicrobien », on entend un composé capable de diminuer la viabilité d'un micro-organisme et/ou d'en diminuer la croissance ou la reproduction. De tels agents antimicrobiens peuvent être des antibiotiques, lorsqu'ils sont dirigés contre des bactéries. Néanmoins, l'invention est applicable à tout type de micro-organisme du type bactérie, levures, moisissures ou parasites et donc, aux agents antimicrobiens correspondants.

De préférence, l'agent antimicrobien sera un antibiotique tel qu'une bêta-lactamine, notamment choisie parmi les pénicillines, les céphalosporines, les céphamycines, les carbapénèmes, les monobactames et en particulier parmi l'ampicilline, l'amoxicilline, la ticarcilline, la piperacilline, la céfalotine, le céfuroxime, la céfoxitine, le céfixime, le céfotaxime, la céftazidime, la ceftriaxone, la cefpodoxime, le céfépime, l'aztreonam, l'ertapénème, l'imipénème, le méropénème et le faropénème.

Les carbapénèmes sont utilisés, notamment, en dernier recours pour lutter contre les bactéries Gram-négatives telles que la famille des Entérobactéries, des *Pseudomonas* et des *Acinetobacter.* Un tel antibiotique sera donc déposé sur la zone d'analyse quand il sera suspecté que le micro-organisme présent est une Entérobactérie ou une autre espèce Gram négative susceptible de présenter une résistance aux carbapénèmes.

Le dépôt est, de préférence, réalisé à partir d'une solution aqueuse de l'agent antimicrobien.

Un tampon adapté à la solubilité de l'agent antimicrobien, ainsi qu'à une activité optimale du mécanisme à l'origine de la résistance ciblée, pourra également être utilisé pour préparer la solution de l'agent antimicrobien. L'ajout de sels de zinc (notamment du type chlorure ou sulfate de zinc) dans la solution antimicrobienne pourra également être envisagée pour une activité optimale des métallo-bêta-lactamases.

Une goutte, par exemple d'environ 1 à 2 microlitres, de la solution antimicrobienne pourra être déposée, de telle sorte que toute la zone d'analyse soit recouverte. L'eau contenue dans la solution est ensuite évaporée, par exemple par simple séchage à l'air ambiant et à température ambiante. La plaque d'analyse peut être laissée à une température appartenant, par exemple, à la gamme allant de 17 à 40°C, et notamment à température ambiante (22°C). Il est également possible de la transférer dans une enceinte thermostatée, par exemple à 37°C, pour accélérer le séchage.

De manière très simple, l'agent antimicrobien sera déposé en solution aqueuse, ce dépôt étant suivi d'une opération de séchage. On obtient ainsi une zone d'analyse porteuse d'un agent antimicrobien, dite fonctionnalisée. Il est également possible que l'agent antimicrobien soit immobilisé sur la zone d'analyse par liaison électrostatique, ionique, covalente ou liaison d'affinité ou grâce à un agent adhésif. Un simple dépôt de l'agent antimicrobien pourra ne pas assurer une immobilisation satisfaisante de ce dernier. En effet, dans ce cas, si l'agent antimicrobien n'adhère pas suffisamment à la zone de caractérisation, cela peut entraîner une perte de l'agent antimicrobien au cours du dépôt de la population de micro-organisme(s), nécessitant ainsi une certaine dextérité de la part du manipulateur lors de la réalisation du dépôt, ou encore une perte de l'agent antimicrobien par décollement du dépôt lors du stockage de la plaque MALDI pour une utilisation ultérieure. Aussi, à la place d'un simple dépôt, l'agent antimicrobien pourra être lié à la zone d'analyse par des liaisons électrostatiques, ioniques, covalentes, avec ou non l'utilisation d'un bras ou d'un partenaire de liaison spécifique (anticorps, protéines recombinantes de phage) ou non, par l'utilisation d'interaction biotine/streptavidine préalablement greffée à la surface de la zone d'analyse et à ragent antimicrobien, ou par tout type de liaison adaptée à la nature de l'agent antimicrobien et à la surface de la zone d'analyse, ou encore grâce à un agent adhésif. Le mode de liaison ou de dépôt sera néanmoins choisi de manière à ne pas gêner l'éventuelle interaction de l'agent antimicrobien avec un micro-organisme, ce qui pourrait avoir pour conséquence de masquer un phénomène de résistance. En particulier, on préfèrera réaliser l'immobilisation de l'agent antimicrobien par utilisation d'un agent adhésif, plutôt que par liaison covalente ou d'affinité pour éviter des changements de conformation de l'agent antimicrobien et assurer une bonne accessibilité de ce dernier au site actif de l'enzyme qui serait générée par le micro-organisme.

Dans le cas de l'utilisation d'un agent adhésif, c'est-à-dire d'un agent qui va adhérer à la plaque et donc améliorer l'immobilisation de l'agent antimicrobien sur cette dernière, on déposera alors un mélange de l'agent adhésif et de l'agent antimicrobien en solution aqueuse. L'agent adhésif sera, notamment, un polymère soluble dans l'eau, A titre d'exemple d'agent adhésif pouvant être utilisé pour l'immobilisation de l'agent antimicrobien sur la ou les zones d'analyse, on peut citer l'heptakis(2,6-di-*O*-méthyl)-β-cyclodextrine. L'agent adhésif sera choisi en fonction de l'agent antimicrobien à immobiliser sur la zone d'analyse. En particulier, il sera sélectionné en fonction de sa masse, de manière à ce que sa présence ne fausse pas la détection ultérieure par MALDI visant à déterminer la présence ou l'absence de l'agent antimicrobien présent et/ou de ses produits de dégradation. L'homme du métier ajustera la quantité d'agent adhésif utilisé qui ne devra pas être trop importante pour assurer l'accessibilité de l'agent antimicrobien par la population de micro-organisme(s) lorsque celle-ci aura été déposée. Par exemple, dans le cas de l'heptakis(2,6-di-*O*-méthyl)-β-cyclodextrine, on pourra choisir un rapport massique heptakis(2,6-di-*O*-méthyl)-β-cyclodextrlne/agent antimicrobien de 1/20 à 1/2, de préférence de 1/10 à 1/5.

La quantité d'agent antimicrobien déposée sur une zone d'analyse sera adaptée par l'homme du métier, en fonction de l'agent antimicrobien en question. En effet, selon le degré d'ionisation de la molécule, une quantité suffisante devra être déposée pour pouvoir détecter en MALDI-TOF le(s) pic(s) correspondante(s) à l'agent antimicrobien avec des intensités au-dessus du bruit de fond. A l'inverse, une quantité trop importante en agent antimicrobien risquerait de masquer la détection du phénomène à l'origine de la résistance à caractériser, de telle sorte que la diminution d'intensité du pic correspondant à l'agent antimicrobien natif ne pourrait pas être observée. L'excès en agent antimicrobien pourrait, notamment, compromettre la détection de betâ-lactamases ayant une faible activité. Par exemple, de 0,04 à 4 g/m² d'agent antimicrobien sera déposé, Pour cela, on déposera notamment une solution dans l'eau ultra-pure à une concentration de 0,1 à 10 mg/ml en agent antimicrobien. A titre d'exemple, pour l'ampicilline, on pourra déposer une solution aqueuse comprenant de 1,7 à 10 mg/ml d'ampicilline et pour le faropénème, on pourra déposer une solution aqueuse comprenant de 0,1 à 1 mg/ml de faropénème.

Une zone de caractérisation sera, de préférence, porteuse d'un seul agent antimicrobien, bien que l'utilisation de plusieurs agents antimicrobiens sur une même zone d'analyse ne soit pas exclue. Une zone de caractérisation porteuse de plusieurs agents antimicrobiens permettrait de tester la présence de plusieurs enzymes en même temps et donc de différents phénomènes de résistance. Dans le cas d'une zone de caractérisation porteuse de plusieurs agents antimicrobiens, ces derniers seront sélectionnés, de manière à ce que la masse de leur forme native et/ou leurs produits de dégradation sous l'action de l'enzyme cible ne soient pas chevauchantes, pour pouvoir les détecter séparément par MALDI-TOF. Il est, par exemple, possible de déposer en plus d'un agent antimicrobien, un autre agent antimicrobien de la même famille ou d'une famille différente. Par exemple, certains carbapénèmes sont plus adaptés pour révéler une carbapénémase particulière. Il est donc possible d'envisager d'avoir plusieurs types de carbapénèmes ou autres bêta-lactamines sur une même zone de caractérisation. Par contre, si deux agents antimicrobiens sont déposés sur une même zone, ils seront choisis de manière à ce que leur spectre d'activité n'interfère pas l'un avec l'autre et qu'il soit détectable distinctement par MALDI-TOF.

Il est également possible de déposer en plus de l'agent antimicrobien, un autre composé. Dans le cas des bêta-lactamines, il est également possible de déposer un inhibiteur des bêta-lactamases, afin de caractériser un phénomène BLSE (bêta-lactamase à spectre étendu), comme mis en oeuvre notamment dans la demande WO 2012/023845. En particulier, on pourra déposer une combinaison d'une bêta-lactamine avec un inhibiteur de bêta-lactamase tel que l'acide clavulanique, le sulbactam ou le tazobactam. Lorsque la plaque MALDI comporte plusieurs zones d'analyse, de manière avantageuse, au moins deux zones, voire plus, seront porteuses d'un agent antimicrobien différent. Il est ainsi possible de caractériser plusieurs populations de micro-organisme(s) avec une même plaque. Chacune des zones pourra être porteuse d'un agent antimicrobien différent. Le plus souvent, cependant, les caractérisations sont effectuées en duplicata, de sorte qu'un même agent antimicrobien sera présent sur au moins deux zones d'analyse, voire plus.

De telles plaques MALDI fonctionnalisées pourront être directement fournies à l'utilisateur, qui n'aura donc plus qu'à y déposer la population de micro-organisme(s) à étudier, puis après une étape d'incubation, la matrice MALDI. Elles pourront être commercialisées, dans un emballage individuel ou comprenant plusieurs plaques.

### Préparation et dépôt de la population de micro-organisme(s)

Dans le cadre de l'invention, une population de micro-organisme(s) est déposée sur une zone d'analyse d'une plaque MALDI fonctionnalisée avec un agent antimicrobien, pour ensuite procéder à sa caractérisation.

La population de micro-organisme(s) pourra provenir de différentes sources. A titre d'exemple de source de micro-organisme(s), on peut citer les échantillons d'origine biologique, notamment animale ou humaine. Un tel échantillon peut correspondre à un prélèvement de fluide biologique, du type sang total, sérum, plasma, urine, liquide céphalo-rachidien, sécrétion organique, à un prélèvement tissulaire ou à des cellules isolées. Ce prélèvement pourra être déposé tel quel, ou sera, de préférence, soumis préalablement au dépôt sur la zone d'analyse concernée, à une préparation de type enrichissement ou culture, à une concentration et/ou à une étape d'extraction ou de purification selon des méthodes connues de l'homme du métier. Néanmoins, une telle préparation ne peut pas correspondre à une étape de lyse qui entraine la désintégration des micro-organismes et une perte de leur contenu avant le dépôt sur la zone d'analyse. La population de micro-organisme(s) pourra être déposée sous la forme d'un inoculum. Dans le cadre de l'invention, la population de micro-organisme(s) déposée sur la zone d'analyse sera, de préférence, une population de micro-organisme(s) vivant(s), bien qu'il ne soit pas exclu de réaliser une extraction de la population de micro-organisme(s) à partir d'un échantillon biologique, en utilisant un détergent ce qui pourra affecter la viabilité, Mais, dans un tel cas, pour un test immédiat de la population de micro-organisme(s) en MALDI, le stock d'enzymes actives déjà présent pourra permettre de réaliser la caractérisation de la population de micro-organisme(s), en détectant l'éventuel phénomène de résistance.

La source de la population de micro-organisme(s) peut également être un produit de l'agro-alimentaire tel que la viande, le lait, le yaourt et tout autre produit consommable susceptible d'être contaminé, ou encore un produit cosmétique ou pharmaceutique. Là encore, un tel produit pourra être soumis a une préparation de type enrichissement ou culture, à une concentration et/ou à une étape d'extraction ou de purification, afin d'obtenir la population de micro-organisme(s) à déposer.

Le plus souvent, la source de micro-organisme(s) pourra au préalable avoir été mise en culture dans un bouillon ou sur une gélose de manière à l'enrichir en micro-organismes. De tels milieux, gélosés ou en bouillon, sont bien connus de l'homme de l'art. L'enrichissement sur gélose est particulièrement favorable puisqu'il permet d'obtenir des colonies de micro-organismes qui pourront directement être déposées sur la zone d'analyse.

Dans le cadre de l'invention, on pourra déposer sur la zone d'analyse, de préférence, un milieu cellulaire comprenant une population bactérienne, et non une ou plusieurs protéines obtenues après une étape d'extraction ou de purification, comme dans le cas des techniques MS/MS ou MRM. De préférence, la population de micro-organismes déposée contient au moins 10⁵ UFC de micro-organismes. A titre d'exemple, on pourra déposer de 10⁵ à 10⁹ UFC d'un micro-organisme. Il est, par exemple, possible de procéder directement au dépôt d'une biomasse, d'une goutte d'une suspension de micro-organismes, dans de l'eau ultra-pure ou un tampon. On pourra déposer une colonie ou une fraction de colonie d'un micro-organisme.

La population déposée, comprendra, de préférence, une seule espèce de micro-organisme. Il n'est cependant pas exclu de déposer sur la zone d'analyse une population comprenant différents micro-organismes. Dans ce cas, il sera préférable que les micro-organismes soient connus pour être susceptibles de développer des mécanismes de résistance différents, de manière à savoir lequel présenterait la résistance qui serait identifiée.

Dans le cadre de l'invention, il n'est pas utile de procéder à une préparation particulière d'un échantillon qui serait déposé, En particulier, la population de micro-organisme(s) est déposée, sans avoir été préalablement mise en contact avec un agent antimicrobien. En effet, dans le cadre de l'invention, il n'est pas nécessaire de procéder à une préparation longue et fastidieuse d'un échantillon à déposer, la population de micro-organisme(s) déposée pouvant être préparée sans étape de centrifugation.

Le dépôt est réalisé de manière à ce que la population de micro-organisme(s) soit déposée de manière homogène sur la zone d'analyse. Pour cela, on pourra utiliser les procédures décrites pour la réalisation de l'identification standard de micro-organismes, dans les manuels d'utilisation des appareillages MALDI-TOF commerciaux, tels que l'appareillage VITEK^{®} MS commercialisé par la société bioMérieux.

Il est néanmoins possible d'ajouter, en plus de la population de micro-organisme(s), un composé connu pour accélérer la réaction enzymatique mise en oeuvre dans le mécanisme de résistance considéré. Ce composé peut par exemple être du zinc, sous la forme ZnCl₂ ou sulfate de zinc notamment, qui est un co-facteur important pour l'activité des métallo-bêta-lactamases. Ce composé peut être déposé au préalable sur la zone d'analyse en combinaison avec l'agent antimicrobien ou à tout autre moment dans la préparation de la zone de caractérisation.

Le fait que ce qui est déposé sur la zone d'analyse contienne bien une population d'au moins un micro-organisme, peut être déterminé au préalable par un test adapté, notamment par le fait qu'il s'agisse d'une colonie isolée sur gélose. De manière préférée, on déposera sur la zone d'analyse une seule population d'un seul micro-organisme.

### Incubation

Après dépôt de la population de micro-organisme(s), la zone d'analyse porteuse à la fois de l'agent antimicrobien et de la population d'un micro-organisme à caractériser, est soumise à une étape d'incubation pour permettre l'interaction entre le micro-organisme et l'agent antimicrobien et donc dans le cas de la présence d'une population de micro-organismes, résistants à l'agent antimicrobien, permettre à la réaction/au phénomène à l'origine de la résistance de se produire. En particulier, lorsque le phénomène de résistance à détecter est dû à la présence d'une enzyme produite par le microorganisme déposé, l'incubation sera réalisée de manière à permettre à la réaction enzymatique de se produire.

Dans le cadre de l'invention, le phénomène responsable de la résistance à un agent antimicrobien a donc lieu directement sur la plaque MALDI et nullement préalablement au dépôt sur la plaque MALDI de la population de micro-organisme(s) déjà mise en présence de l'agent antimicrobien, comme dans les techniques antérieures. Le phénomène responsable de la résistance à un agent antimicrobien se produit dans un volume minimal correspondant à la zone de caractérisation. Il y a ainsi limitation des problèmes de dilution rencontrés dans les techniques antérieures.

Les conditions et le temps d'incubation seront adaptés par l'homme du métier en fonction du phénomène de résistance à caractériser.

La plaque d'analyse peut être laissée à une température appartenant, par exemple, à la gamme allant de 17 à 40°C, et notamment à température ambiante (22°C). Il est également possible de la transférer dans une enceinte thermostatée, par exemple à 37°C, pour favoriser la réaction enzymatique qui pourrait être à l'origine du phénomène de résistance.

Les conditions d'humidité seront adaptées pour éviter le dessèchement des micro-organismes présents, en particulier quand le phénomène de résistance à détecter met en oeuvre une réaction d'hydrolyse. La plaque pourra être placée pendant l'incubation dans une atmosphère humide, à moins que le taux d'humidité apporté directement par le dépôt contenant la population de micro-organisme(s) soit suffisant.

Dans les conditions sélectionnées, le temps d'incubation devra être suffisant pour permettre la détection ultérieure par MS MALDI-TOF du phénomène de résistance à détecter, notamment de la réaction enzymatique dans le cas de phénomènes de résistance médiés par une enzyme. L'incubation est, le plus souvent, réalisée pendant au moins 5 minutes, plus préférentiellement pendant au moins 20 minutes, et encore plus préférentiellement pendant une durée de 45 à 90 minutes.

Il a été constaté, dans le cadre de l'invention, que la réalisation d'une telle étape d'incubation ne gênait en rien une identification ultérieure du micro-organisme, s'il était souhaité de réaliser une telle caractérisation, en plus de la détection du phénomène de résistance.

### Dépôt de la matrice MALDI

Généralement, les matrices utilisées dans la technique MALDI sont photosensibles et cristallisent en présence de la population de micro-organisme(s), tout en préservant l'intégrité des molécules et micro-organismes présents. De telles matrices, notamment adaptées à la technique SM MALDI-TOF, sont bien connues et, par exemple, constituées à partir d'un composé choisi parmi : l'acide 3,5-diméthoxy-4-hydroxycinnamique; l'acide α-cyano-4-hydroxycinnamique, l'acide férulique et l'acide 2,5-dihydroxybenzoïque, De nombreux autres composés sont connus de l'homme du métier. Il existe également des matrices liquides qui ne cristallisent ni à pression atmosphérique, ni même sous pression. Tout autre composé qui permettra d'ioniser les molécules présentes dans la zone de caractérisation sous l'effet d'un rayon laser pourra être utilisé.

Pour la constitution de la matrice, un tel composé est mis en solution, le plus souvent dans l'eau, de préférence de qualité « ultra-pure », ou dans un mélange eau/solvant(s) organique(s). A titre d'exemple de solvants organiques classiquement utilisés, on peut citer, l'acétone, l'acétonitrile, le méthanol ou l'éthanol. L'acide trifluoroacétique (TFA) peut parfois être ajouté. Un exemple de matrice est, par exemple, constituée de 20 mg/mL d'acide sinapique dans un mélange acétonitrile/eau/TFA de 50/50/0,1 (v/v). Le solvant organique permet aux molécules hydrophobes présentes de se dissoudre dans la solution, alors que l'eau permet la dissolution des molécules hydrophiles. La présence d'acide, tel que le TFA, favorise l'ionisation des molécules par captation d'un proton (H⁺).

La solution constitutive de la matrice est directement déposée sur la zone d'analyse et recouvre alors la population de micro-organisme(s) et l'agent antimicrobien présents sur cette dernière.

De façon optionnelle, le procédé selon l'invention contient, en outre, avant l'étape d'ionisation de la zone de caractérisation, une étape de cristallisation de la matrice présente. Le plus souvent, la cristallisation de la matrice est obtenue en laissant sécher la matrice à l'air ambiant. Le solvant présent dans la matrice est ainsi évaporé, par exemple, en laissant la plaque d'analyse à une température appartenant, par exemple, à la gamme allant de 17 à 30°C, et notamment à température ambiante (22°C) pendant quelques minutes, par exemple de 5 minutes à 2 heures. Cette évaporation du solvant permet la cristallisation de la matrice dans laquelle la population de micro-arganisme(s) et l'agent antimicrobien sont répartis.

### Ionisation et obtention du spectre de masse

La population de micro-organisme(s) et l'agent antimicrobien, placés au sein de la matrice MALDI, formant la zone de caractérisation, sont soumis à une ionisation douce.

Le rayon laser utilisé pour l'ionisation pourra avoir tout type de longueur d'onde favorable à la sublimation ou à la vaporisation de la matrice. De préférence, une longueur d'onde ultraviolette ou même infrarouge sera utilisée. Cette ionisation pourra, par exemple, être réalisée avec un laser à azote émettant un rayon UV à 337.1 nm.

Lors de l'ionisation, la population de micro-organisme(s) et l'agent antimicrobien sont soumis à une excitation par laser, La matrice absorbe alors l'énergie photonique et la restitution de cette énergie entraîne la sublimation de la matrice, la désorption des molécules présentes dans la population de micro-organisme(s) et de l'agent antimicrobien et l'apparition de matière dans un état qualifié de plasma. Au sein de ce plasma, il se produit des échanges de charges entre molécules de matrice, des micro-organismes et d'agent antimicrobien. Par exemple, des protons peuvent être arrachés à la matrice et transférés aux protéines, peptides et composés organiques présents au niveau de la zone de caractérisation. Cette étape permet une ionisation douce des molécules présentes sans induire leur destruction. La population de micro-organisme(s) et l'agent antimicrobien libèrent ainsi des ions de différentes tailles. Ces derniers sont alors accélérés par un champ électrique et volent librement dans un tube sous pression réduite, nommé tube de vol. La pression appliquée lors de l'ionisation et lors de l'accélération des ions générés appartient le plus souvent à la gamme allant de 10⁻⁶ à 10⁻⁹ millibars [mbar]. Les ions les plus petits vont alors « voyager » plus rapidement que les ions plus gros permettant ainsi leur séparation. A l'extrémité terminale du tube de vol est situé un détecteur. Le temps de vol (ou TOF pour « *Time Of Flight* » en anglais) mis par les ions est utilisé pour calculer leur masse. Ainsi, un spectre de masse est obtenu, représentant l'intensité du signal correspondant au nombre de molécules ionisées d'une même masse sur charge [m/z], en fonction du rapport m/z des molécules qui frappent le détecteur. Le rapport masse sur charge [m/z] est exprimé en Thomson [Th]. Une fois introduite dans le spectromètre de masse, le spectre d'une zone de caractérisation est obtenu très rapidement, le plus souvent en moins d'une minute.

Un procédé de spectrométrie de masse MALDI-TOF utilisable selon l'invention peut notamment comprendre les étapes successives suivantes pour l'obtention du spectre de masse :
- disposer d'une zone de caractérisation comprenant la population de micro-organisme(s) à étudier et au moins un agent antimicrobien dans une matrice adaptée à la spectrométrie MALDI,
- éventuellement obtenir la cristallisation de la matrice dans laquelle la population de micro-organisme(s) et l'agent antimicrobien sont disposés,
- ioniser le mélange population de micro-organisme(s)/agent antimicrobien/matrice, grâce à un rayon laser,
- accélérer les molécules ionisées obtenues grâce à une différence de potentiel,
- laisser se déplacer librement les molécules ionisées et accélérées dans un tube sous pression réduite,
- détecter au moins une partie des molécules ionisées en sortie du tube, de manière à mesurer le temps qu'elles ont mis pour parcourir le tube sous pression réduite et à obtenir un signal correspondant au nombre de molécules ionisées atteignant le détecteur à un instant donné,
- calculer le rapport masse sur charge [m/z] des molécules détectées, de manière à obtenir un signal correspondant au nombre de molécules ionisées d'une même masse sur charge [m/z], en fonction du rapport m/z des molécules détectées.

En général, le calcul du rapport m/z est obtenu, en tenant compte d'une calibratlon préalable du spectromètre de masse utilisé, sous la forme d'une équation liant le rapport masse sur charge [m/z] et le temps de parcours dans le tube sous pression réduite des molécules ionisées.

La calibration consiste à utiliser une molécule ou un micro-organisme (selon la caractérisation) qui va fournir des molécules ionisées couvrant la gamme de masse correspondant à la caractérisation envisagée. Les rapports m/z de ces molécules ionisées vont servir de standards, afin de permettre à l'appareillage de mesurer les masses de manière adéquate.

Pour l'identification du micro-organisme, la calibration pourra être réalisée à partir d'une souche de bactéries possédant des molécules ionisées ayant des rapports m/z couvrant la gamme de masses utilisées pour l'identification (typiquement dans la gamme allant de 2000 à 20000 Da dans le cas des levures, moisissures, bactéries ou parasites). Pour détecter les signaux correspondants à l'agent antimicrobien, la calibration pourra être réalisée à partir d'un mélange de peptides de petites masses. Dans le cadre de l'invention, le calibrant pepMIX 6 (LaserBio Labs) couvrant une gamme de masse de 350 à 1000 Daltons pourra, par exemple, être utilisé.

Tout type de spectromètre de masse MALDI-TOF peut être utilisé pour l'élaboration du spectre de masse. De tels spectromètres comprennent :
*i)* une source d'ionisation (en général, un laser UV) destinée à ioniser le mélange population de micro-organisme(s)/agent antimicrobien/matrice ;
*ii)* un accélérateur des molécules ionisées par application d'une différence de potentiel ;
*iii)* un tube sous pression réduite dans lequel se déplacent les molécules ionisées et accélérées ;
*iv*) un analyseur de masse destiné à séparer les ions moléculaires formés, en fonction de leur ratio masse sur charge (m /z) ;
*v*) un détecteur destiné à mesurer le signal produit directement par les ions moléculaires.

Dans le cadre de l'invention, l'analyse par MALDI-TOF est, de préférence, une analyse par MALDI-TOF simple, bien qu'une analyse par MALDI-TOF TOF ne soit pas exclue. Une analyse par MALDI TOF-TOF, bien que plus complexe, pourra être envisagée notamment pour améliorer la sensibilité de détection, dans certains cas et mettra en oeuvre un appareillage adapté à une telle analyse.

### Détection d'une résistance à un agent antimicrobien

Par « résistance », on entend un phénomène selon lequel un micro-organisme ne présente pas une diminution de sa viabilité, ou une diminution de sa croissance ou de sa reproduction, quand il est exposé à une concentration d'un agent antimicrobien qui est reconnue comme étant efficace vis-à-vis dudit micro-organisme en l'absence de résistance.

L'identification d'un mécanisme de résistance, à partir du spectre de masse obtenu pour une zone de caractérisation considérée, peut se faire par la détection, sur le spectre de masse obtenu, d'un pic de masse donné, ou d'un changement de pic de masse par rapport à un spectre de masse de référence, notamment, par rapport au spectre de masse de l'agent antimicrobien présent au niveau de ladite zone de caractérisation.

Dans le cadre de l'invention, il a été démontré que la réalisation d'une spectrométrie de masse par MALDI-TOF directement sur un micro-organisme en présence d'un agent antimicrobien permettait de détecter les molécules d'intérêt pertinentes pour la détermination d'une résistance audit agent antimicrobien. La détermination de la résistance éventuelle d'une population d'un micro-organisme pourra donc comprendre les étapes suivantes :
a1) disposer d'un spectre de masse de référence, par exemple pour l'agent antimicrobien et/ou pour ses produits de dégradation ;
   de tels produits de dégradation seront issus du phénomène de résistance et seront notamment dus à la présence d'une enzyme de dégradation,
b1) soumettre la population de micro-organisme(s) et l'agent antimicrobien déposés sur la zone d'analyse et mis en présence de la matrice (correspondant à une zone de caractérisation selon l'invention), à une ionisation,
c1) acquérir un spectre de masse obtenu suite à cette ionisation, dans la gamme de masse d'intérêt pour la détermination de la résistance,
d1) comparer le spectre de masse obtenu à l'étape c1) avec le spectre de référence et en déduire la présence ou non d'une résistance.

A l'étape d1) par exemple, si sur le spectre de masse obtenu à l'étape c1), le ou les pic(s) de masse caractéristique(s) de l'agent antimicrobien ont disparu et/ou si un ou des pic(s) de masse caractéristique(s) d'un ou plusieurs produits de dégradation de l'agent antimicrobien est(sont) présent(s), on en déduit la présence d'un micro-organisme résistant à l'agent antimicrobien. L'interprétation pourra être réalisée, par exemple à partir du rapport d'intensité entre un pic de masse caractéristique de l'agent antimicrobien ou d'un de ses produits de dégradation et un pic de masse caractéristique d'un calibreur externe ou à partir du rapport d'intensité entre un pic de masse caractéristique de l'agent antimicrobien et un pic de masse caractéristique d'un produit de dégradation de l'agent antimicrobien.

Il sera également possible de comparer, le niveau d'intensité entre le ou les pics de masse caractéristique(s) de l'agent antimicrobien ou le ou les pic(s) de masse caractéristique(s) d'un ou plusieurs produits de dégradation de l'agent antimicrobien et un ou plusieurs pics de référence d'un composé présent qui n'est pas soumis aux variations induites par l'activité biologique/enzymatique à tester. Pourront, par exemple, être considérés comme pics de référence, un ou plusieurs pics de la matrice MALDI, un ou plusieurs pics d'un peptide ou d'une molécule de référence ajouté(e) à la matrice ou préalablement séché(e) sur la zone d'analyse, un ou plusieurs pics qui correspondrait à une molécule du microorganisme présent (par exemple, un métabolite) qui serait toujours présente et invariable chez plusieurs espèces.

Dans le cas de la détermination de la résistance, une calibration est réalisée dans une gamme de masse correspondant à des faibles masses, typiquement dans la gamme allant de 200 à 1200 Da, et de préférence dans la gamme allant de 200 à 600 Da. Le spectre de masse obtenu à l'étape c1) est également compris dans cette gamme de masse. Pour réaliser cette calibration, 2 microlitres d'une solution calibrante composée d'un mélange de peptides (pepMIX6, LaserBio Labs) et de matrice HCCA, l'acide α-cyano-4-hydroxycinnamique, pourront, par exemple, être déposés sur une zone d'analyse de référence. Préalablement à l'ionisation des zones de caractérisation, une ionisation du calibrant sur cette zone d'analyse de référence sera réalisée. Les rapports m/z des molécules ionisées du calibrant serviront alors de standards afin de permettre à l'appareillage de mesurer les masses de manière adéquate.

Le procédé selon l'invention pourra notamment être mis en oeuvre pour détecter une résistance due à la capacité d'un micro-organisme à sécréter une enzyme connue pour dégrader les antibiotiques du type bêta-lactamines, et notamment choisies parmi les pénicillinases, les céphalosporinases, les céphamycinases, les carbapénémases. L'invention est également adaptée à la détection d'autres phénomènes de résistance basés sur une dégradation ou une modification enzymatique entraînant un changement de masse de l'agent antimicrobien, A titre d'exemple, on peut citer des mécanismes de résistance tels que la dégradation des macrolides par des estérases ou la dégradation de la fosfomycine par les époxidases, l'acétylation des aminosides, du chloramphénicol ou encore des streptogramines, la phosphorylation des aminosides, des macrolides, de la rifampicine et des antibiotiques peptidiques, l'hydroxylation de la tétracycline, l'adénylation des aminosides et des lincosamides, l'ADP-ribosylation de la rifampicine, et la glycosylation des macrolides et de la rifampicine.

Le terme « produit de dégradation » inclut tout produit correspondant à une modification de la structure chimique de l'agent antimicrobien, du fait de l'action du microorganisme présent. Il peut, en plus des mécanismes de dégradation et de modification enzymatique mentionnés ci-dessus, s'agir également de rajout d'un groupement détectable en MALDI-TOF qui inactive l'agent antimicrobien ou qui l'empêche de se fixer sur sa cible.

Un tel procédé de détection de la résistance pourra être mis en oeuvre avec des plaques MALDI préalablement fonctionnalisées, en faisant appel aux dispositifs MALDI-TOF disponibles dans le commerce. Seule la calibration et l'étape d'interprétation devront être adaptées pour permettre la détection de la résistance. La détection de la résistance aux agents antimicrobiens, et en particulier à un antibiotique donné, dont la détermination rapide en routine peut être vitale dans de nombreux cas cliniques, est maintenant possible dans le cadre de l'invention.

Le procédé de caractérisation selon l'invention, qui permet l'indentification de la présence d'un micro-organisme résistant aux antibiotiques, dans un temps très court, présente un intérêt tout particulier pour mener un diagnostic rapide. C'est en particulier le cas pour la détection d'Enterobactéries productrices de carbapénémase (CPE). Le procédé selon l'invention permettra de réaliser des tests rapides en milieu hospitalier, afin d'adapter rapidement le traitement antibiotique administré.

### Identification d'un micro-organisme

Les micro-organismes qui peuvent être identifiés par le procédé de l'invention sont tout type de micro-organismes, pathogènes ou non, rencontrés tant dans l'industrie que dans la clinique, qui peuvent connaître des phénomènes de résistance aux agents antimicrobiens, Ce peut être, de préférence, des bactéries, des moisissures, des levures ou des parasites. L'invention est particulièrement adaptée à l'étude des bactéries, A titre d'exemple de tels micro-organismes, on peut citer les bactéries Gram-positives, Gram-négatives et les *Mycobacteria*. A titre d'exemple de bactéries Gram-négatives, on peut citer celles des genres : *Pseudomonas, Escherkhia, Salmonella, Shigella, Enterobacter, Klebsiella, Serratia, Proteus, Acinetobacter, Gimbacter, Aeromonas, Stenotrophomonas, Morganella, Enterococcus* et *Providencia, et notamment Escherichia coll, Enterobacter cloacae, Enterobacter aerogenes, Citrobacter sp*., *Klebsiella pneumoniae, Klebsiella oxytoca, Pseudomonas aeruginosa, Providencia rettgeri, Pseudomonas putida, Stenotrophomonas maltophilia, Acinetobacter baumanii, Comamonas sp., Aeromonas sp., Morganella morganii, Enterococcus sp*., *Proteus mirabilis, Saimonella senftenberg, Serratia marcescens, Salmonella typhimurium etc.,* A titre d'exemple de bactéries Gram-positives, on peut citer celles des genres : *Enterococcus, Streptococcus, Staphylococcus, Bacillus, Listeria et Clostridium.*

Des spectres de référence obtenus par MALDI-TOF pour de tels micro-organismes correspondant à leurs protéines majoritaires, sont disponibles et enregistrés dans des bases de données disponibles avec les appareils MALDI-TOF commerciaux, et permettent, par comparaison, l'identification de la présence de tels micro-organismes.

L'identification d'une population d'un micro-organisme présent pourra donc comprendre les étapes suivantes :
a2) disposer d'un spectre de masse de référence, pour au moins un micro-organisme et le plus souvent pour une série de micro-organismes,
b2) soumettre la population de micro-organisme(s) et l'agent antimicrobien déposés sur la zone d'analyse et mis en présence de la matrice (correspondant à une zone de caractérisation selon l'invention), à une ionisation,
c2) acquérir un spectre de masse obtenu suite à cette ionisation, dans la gamme de masse d'intérêt pour l'identification du micro-organisme,
d2) comparer le spectre de masse obtenu à l'étape c2) avec le spectre de référence et en déduire la famille, le genre, ou, de préférence, l'espèce d'au moins un micro-organisme.

Dans le cas de l'identification de micro-organismes, une calibration est réalisée dans une gamme de masse correspondant à des masses élevées, typiquement dans la gamme allant de 2000 à 20000 Da, et de préférence dans la gamme allant de 3000 à 17000 Da. Le spectre de masse obtenu à l'étape c2) est également compris dans cette gamme de masse.

La calibration pourra être effectuée en plaçant une population d'un micro-organisme de référence dans une zone d'analyse de référence présente sur la plaque et en procédant à son analyse par MALDI-TOF. Un tel micro-organisme de référence pourra être, par exemple, une bactérie *E Coli.* Pour cette calibration, on pourra utiliser les procédures décrites pour la réalisation de l'identification standard de micro-organismes, dans les manuels d'utilisation des appareillages MALDI-TOF commerciaux, tels que l'appareillage VITEK^{®} MS commercialisé par la société bioMérieux.

Il est possible d'utiliser deux zones d'analyse de référence pour la calibration : une pour l'identification du micro-organisme et une autre pour la caractérisation de la résistance. Il est également possible d'utiliser la même zone de référence pour faire les deux calibrations. Dans un tel cas, la zone de référence sera fonctionnalisée avec l'agent antimicrobien pour lequel la résistance est à étudier.

Dans le cadre de l'invention, de manière préférée, mais non obligatoire, lorsque deux caractérisations : détection de la résistance et identification d'un micro-organisme seront mises en oeuvre, la détection de la résistance sera réalisée après, c'est-à-dire que les étapes d'ionisation et d'analyse nécessaires à cette détection de la résistance seront menées sur la zone de caractérisations après celles nécessaires à l'identification du micro-organisme.

Lorsque deux caractérisations d'une même zone de caractérisation sont effectuées, elles peuvent être menées sans enlever la plaque d'analyse du spectromètre de masse utilisé pour l'analyse MALDI-TOF. Cette double caractérisation pourra donc comprendre les étapes suivantes :
a3) disposer d'un spectre de masse de référence 1 pour aux moins un micro-organisme et le plus souvent pour une série de micro-organismes ainsi qu'un spectre de masse de référence 2 pour l'agent antimicrobien et/ou pour ces produits de dégradation,
b3) effectuer une calibration du spectromètre de masse utilisé grâce au calibrant utilisé pour l'identification et qui a été déposé au préalable sur la zone d'analyse de référence,
c3) soumettre la population de micro-organisme(s) et l'agent antimicrobien déposés sur la zone d'analyse et mis en présence de la matrice (correspondant à une zone de caractérisation selon l'invention), à une ionisation,
d3) acquérir un spectre de masse obtenu suite à cette ionisation, dans la gamme de masse d'intérêt pour l'identification du micro-organisme,
e3) effectuer une deuxième calibration du spectromètre de masse utilisé grâce au calibrant utilisé pour la détection de la résistance et qui a été déposé au préalable sur une zone d'analyse de référence,
f3) resoumettre la population de micro-organisme(s) et l'agent antimicrobien déposés sur la zone d'analyse et mis en présence de la matrice (correspondant à une zone de caractérisation selon l'invention), à une ionisation,
g3) acquérir un spectre de masse obtenu suite à cette ionisation, dans la gamme de masse d'intérêt pour la détermination de la résistance,
h3) comparer le spectre de masse obtenu à l'étape d3) avec le ou les spectres de référence 1 et en déduire la famille, le genre, ou, de préférence, l'espèce d'au moins un micro-organisme présent.
i3) comparer le spectre de masse obtenu à l'étape g3) avec le spectre de référence 2 et en déduire la présence ou non d'une résistance.

Les étapes c3) et f3) sont donc réalisées sur la même zone de caractérisation et donc sur la même population de micro-organisme(s), ce qui permet donc de réduire les étapes de préparation et de manipulation.

Dans le cadre de l'invention, la même population de micro-organisme(s) et un seul dépôt de cette dernière sur une seule zone d'analyse sont utilisés pour conduire à une zone de caractérisation soumise successivement à deux ionisations permettant d'obtenir les deux caractérisations (caractérisation d'un phénomène de résistance d'un micro-organisme à un agent antimicrobien et identification d'un micro-organisme).

En effet, il a été mis en évidence, de manière inattendue, que la présence de l'agent antimicrobien et la réalisation d'une étape d'incubation préalable, nécessaires pour la détection du phénomène de résistance, ne gênaient en rien la possibilité d'identification du micro-organisme.

Les étapes de comparaison h3) et i3) pourront être menées à la fin du procédé ou après chaque acquisition concernée.

De manière préférée, la population déposée correspondra à une population d'un seul micro-organisme et les deux caractérisations : détection de la résistance et identification d'un micro-organisme, concerneront le même micro-organisme.

En conclusion, l'invention permet :
- l'obtention rapide d'informations à partir d'une même zone de caractérisation, et en particulier à la fois la caractérisation d'un phénomène de résistance à un agent antimicrobien et l'identification d'un micro-organisme,
- une simplification des méthodes d'analyse par MALDI-TOF, sans préparation préalable longue et fastidieuse d'un échantillon avant dépôt sur plaque MALDI pour la caractérisation d'un phénomène de résistance à un agent antimicrobien,
- un contact optimum directement sur la plaque MALDI du micro-organisme et de l'agent antimicrobien, ayant un impact positif dans certain cas sur la sensibilité de détection du phénomène de résistance,
- une réduction du temps et du coût en consommable et en opérations manuelles pour réaliser la caractérisation d'un phénomène de résistance à un agent antimicrobien, par MALDI-TOF,
- une meilleure traçabilité et une meilleure gestion des échantillons à analyser, étant donné qu'aucune incubation préalable en présence d'un agent antimicrobien n'est nécessaire avant son dépôt sur plaque MALDI.

Les exemples ci-après permettent d'illustrer l'invention mais n'ont aucun caractère limitatif. Les analyses ont été effectuées avec l'appareillage VITSK^{®} MS commercialisé par la société bioMérieux. Les analyses ont été réalisées, dans chacun des exemples ci-après, à la fin de l'acquisition. Les spectres ont été acquis sur toutes les zones de caractérisation et ensuite analysés :
- pour l'indentification, l'analyse du spectre a été réalisée à l'aide du moteur de calcul VITEK MS et de la base de données V2.0.0 contenue dans le logiciel Spectra Identifier Version : 2.1.0,
- pour l'hydrolyse, les pics des spectres ont été visualisés à l'aide du logiciel d'acquisition Launchpad V2.8.

### Exemple 1

Des expériences préalables ont été menées pour montrer que la détection par MALDI-TOF de pics d'antibiotique du type bêta-lactamines après dépôt sur une zone analyse d'une plaque MALDI, avec une goutte d'une solution contenant l'antibiotique mélangé avec une matrice appropriée à la technique MALDI, était possible. Dans la présente expérience, une zone d'analyse d'une plaque MALDI (VITEK^{®} MS disposable slides TO-430) a été traitée avec un antibiotique bêta-lactamine et testée pour évaluer la capacité de l'antibiotique à être clivé par une bêta-lactamase. L'ampicilline a été utilisée comme modèle d'antibiotique bêta-lactamine. L'expérience a été réalisée en mettant en oeuvre les étapes ci-dessous :
- 2 µl d'une solution d'ampicilline à 10 mg/ml dans de l'eau, ont été déposés sous la forme d'une goutte, sur une zone d'analyse de la plaque MALDI. La plaque a été incubée à 37°C, jusqu'à ce que la goutte soit complètement sèche.
- 2 µl d'une bêta-lactamase recombinante (β lactamase de *Pseudomonas aeruginosa,* Sigma-Aldrich Lot L6170-550UN. CAS : 9073-60-3) à 1mg/ml en eau ont été utilisés diluée dans de l'eau supplémentée avec du zinc sous la forme de sulfate de zinc (0,76 mM) qui favorise l'activité enzymatique, ont été déposés sur la zone d'analyse sèche porteuse de l'ampicilline. Un contrôle négatif a également été réalisé en parallèle, en déposant sur une autre zone d'analyse traitée de la même manière avec l'ampicilline, une goutte de 2 µl d'eau avec 0,76 mM de zinc, mais sans enzyme.
- Pour que la réaction enzymatique ait lieu, la plaque a été incubée à température ambiante pendant une heure.
- 1 µl d'une matrice HCCA, acide alpha-cyano 4 hydroxycinnamique (VITEK MS matrice CHCA, bioMérieux Ref : 411071), a été déposé sur les zones d'analyses contenant déjà l'ampicilline avec ou sans bêta-lactamase recombinante.
- Une analyse par spectromètre de masse MALDI-TOF a été réalisée avec un appareillage VITEK^{®} MS, en utilisant 2 µl d'un mélange d'HCCA et de pepMIX 6 (acheté chez LaserBio Labs) utilisé en tant que calibrant, qui ont été déposés sur une zone d'analyse de référence, pour la calibration de l'appareillage pour les faibles masses. Ce mélange a été préparé en ajoutant un volume de pepMix6 (10X) à 9 volumes de HCCA. La solution de pepMix6 (10X) a été préparée dans du diluant (acide trifluoroacétique 0,01% dans de l'eau ultrapure), selon les recommandations du fournisseur. Les pics ont été acquis pour une gamme de faible masse allant de 200 à 1200 Da et la présence de molécules correspondant à la molécule native d'ampicilline ou à ses sous-produits de dégradation suite à l'hydrolyse, a été étudiée.

Tous les échantillons ont été analysés en duplicata sur la plaque.

La **Figure 2** présente les spectres de masse obtenus et montre que l'ampicilline n'est pas stable sur la plaque et qu'elle est efficacement dégradée par la bêta-lactamase après incubation. En effet, sur la **Figure 2**, on observe un pic majoritaire à 368,09 m/z correspondant à la forme hydrolysée de l'ampicilline (masse calculée de la forme monosodique de l'amplcilline correspondant à 368,4 m/z) et une perte complète de la forme native à 372,09 m/z (masse calculée de la forme monosodique de l'ampicilline correspondant à 372,4 m/z) sur le spectre du haut correspondant à la zone analysée avec la bêta-lactamase. Par contre, pour le contrôle négatif (spectre du bas), le pic majoritaire est à 372,09 m/z, ce qui correspond à la forme native monosodique de l'ampicilline. Une légère hydrolyse spontanée de l'ampicilline est également constatée dans le contrôle négatif. Cela démontre que la réaction enzymatique d'hydrolyse des bêta-lactamines peut être détectée par la technique MALDI-TOF, par utilisation du procédé de préparation de la zone de caractérisation selon l'invention.

### Exemple 2.

Cet essai a été réalisé pour étudier la dégradation de l'ampicilline par différentes souches de bactéries, après dépôt direct de colonies sur des zones d'analyse porteuses d'ampicilline, après dépôt d'une solution de cette dernière et séchage, comme décrit à l'exemple 1.
Les souches de bactéries et leurs caractéristiques sont présentées dans le **Tableau 1** ci-dessous.

**Tableau 1**

| Souche de bactéries/ N° de référence | Espèces | | CMI*d'ampicilline | Phénotype | Facteur de virulence** |
|---|---|---|---|---|---|
| 1 | | K. *pneumoniae* | 32 | résistant | ----- |
| 2 | | E. *coli* | >128 | résistant | pénicillinase |
| 3 | | E. *coli* | > 128 | résistant | TEM β-lactamase |
| 4 | | E. *coli* ATCC 25922 | 4 | sensible | pas de β-lactamase |
| *CMI : concentration minimale inhibitrice de la souche | | | | | |
| **Facteur de virulence : correspond à l'enzyme sécrétée connue | | | | | |

Les essais ont été réalisés en mettant en oeuvre les étapes ci-dessous :
- 2 µl d'une solution aqueuse d'ampicilline à 10 mg/ml, supplémentée avec du zinc (0,76 mM), ont été déposés sur les zones d'analyse de la plaque MALDI (conforme à celle de l'exemple 1).
- La plaque a été incubée à 37°C, jusqu'à ce que les gouttes de la solution déposée soient complètement sèches.
- Pour chaque souche de bactéries, une portion de colonie obtenue suite à une croissance pendant 24h sur un milieu gélosé a été déposée en duplicata sur des zones d'analyse porteuses de l'ampicilline. Chaque spot a été obtenu, comme décrit dans la procédure VITEK^{®} MS pour la réalisation d'identification de micro-organismes.
- La plaque a été incubée à température ambiante (20-25°C) pendant une heure dans une atmosphère humide. Pour cela, une enceinte fermée contenant de l'eau a été utilisée comme chambre d'incubation pour la plaque.
- 1 µl d'une matrice d'HCCA a été ajouté sur les zones d'analyse porteuses, à la fois, de l'antibiotique et des bactéries.
- Une analyse par spectre de masse MALDI-TOF a été réalisée avec un appareillage VITEK^{®} MS, avec mise sous vide de la plaque, une fois placée dans la chambre de l'appareillage, en menant les deux étapes ci-dessous :
   ∘ une première acquisition du spectre des zones de caractérisation" après calibration de l'appareillage à partir d'une zone de référence porteuse d'une souche *E. coli* (E-cal) déposée,
   ∘ une seconde acquisition des mêmes zones de caractérisation, après une calibration de l'appareillage sur les faibles masses avec le pepMIX 6, en tant que calibrant ; cette deuxième acquisition a été réalisée immédiatement après la première, sans casser le vide de la chambre, pas plus qu'en enlevant la plaque de l'appareillage.
- Les données ont été accumulées et comparées avec celles contenues dans la base de données pour l'identification de l'espèce.
- Les pics ont été visualisés pour une gamme de faibles masses pour la détection de la forme native ou hydrolysée de l'antibiotique.

Tous les échantillons ont été analysés en duplicata sur la plaque.

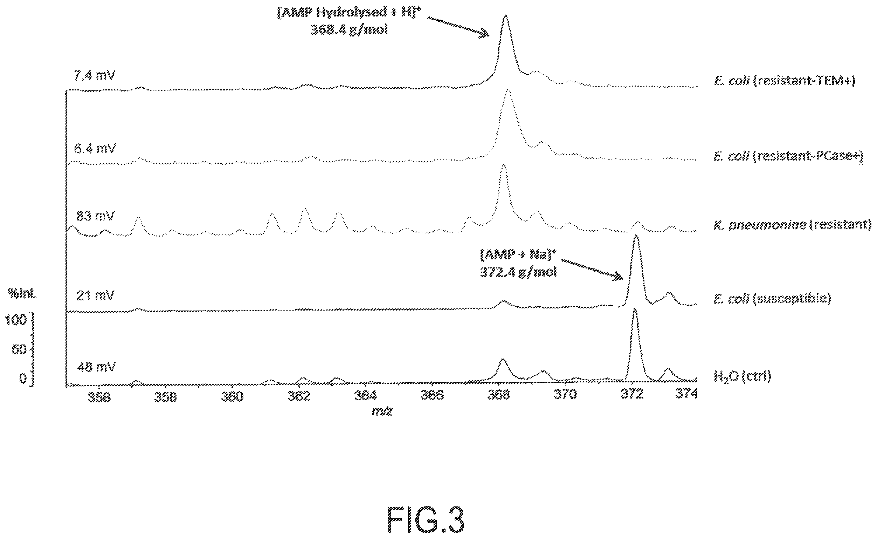

La **Figure 3** présente les spectres de masse obtenus lors de la deuxième série d'acquisitions et montre que les souches résistantes à l'ampicline ont toutes été capables d'hydrolyser l'ampicilline dans les conditions expérimentales utilisées, alors qu'aucune dégradation de l'ampicilline n'a été observée dans le cas de la souche *E. coli* ATCC 25922, connue pour être sensible à l'ampicilline. En effet, après la seconde acquisition dans les faibles masses, un pic majoritaire à 372,15 m/z correspondant à la forme native de l'ampicilline a été observé pour la souche sensible et le contrôle négatif alors que pour toutes les souches résistantes à l'ampicilline, le pic majoritaire était situé à 368,15 m/z correspondant à la forme hydrolysée de l'ampicilline et à la disparition de la forme native.

Après la première acquisition et l'obtention des spectres correspondants, toutes les souches de bactéries ont pu être identifiées avec un niveau de confiance élevé.

Comme présenté dans le **Tableau 2** ci-dessous, une identification correcte des bactéries par MALDI-TOF a pu être obtenue à partir de la première série d'acquisitions pour toutes les zones de caractérisation, avec un niveau de confiance variant de 99,9 à 100%, montrant que les conditions expérimentales permettent encore de discriminer les différentes espèces.

**Tableau 2**

| **Souche de bactéries/ N° de référence** | **Nombre de pics utilisés par l'identification** | **Espèces** | **Probabilité après comparaison des spectres de référence** |
|---|---|---|---|
| 1 | 109 | *K. pneumoniae* | 99,9 |
| 1 | 93 | *K. pneumonniae* | 100 |
| 2 | 95 | *E. coli* | 100 |
| 2 | 103 | *E. coli* | 100 |
| 3 | 91 | *E. coli* | 99,9 |
| 3 | 101 | *E. coli* | 99,9 |
| 4 | 94 | *E. coli* | 99,9 |
| 4 | 112 | *E. coli* | 99,9 |

### Exemple 3.

D'autres essais ont été menés pour démontrer que l'identification d'espèce et la détection de production de carbapénémase, à partir d'une même zone de caractérisation, étaient possibles. Pour cela, le faropénème a été utilisé en tant que modèle d'antibiotique carbapénème, et un protocole identique à celui utilisé dans l'exemple 2 a été utilisé. Une solution de faropénème a été préparée avec une concentration en faropénème de 0,5 mg/ml.

Les souches de bactéries utilisées sont détaillées dans le **Tableau 3** ci-dessous.

**Tableau 3**

| **Souche de bactéries/ N° de référence** | **Espèces** | **Phénotype** | **Facteur de virulence** |
|---|---|---|---|
| 5 | *S. marcescens* | résistant | IMP-1 carbapénémase |
| 6 | *E. coli* ATCC 25922 | sensible | pas de B-lactamase |

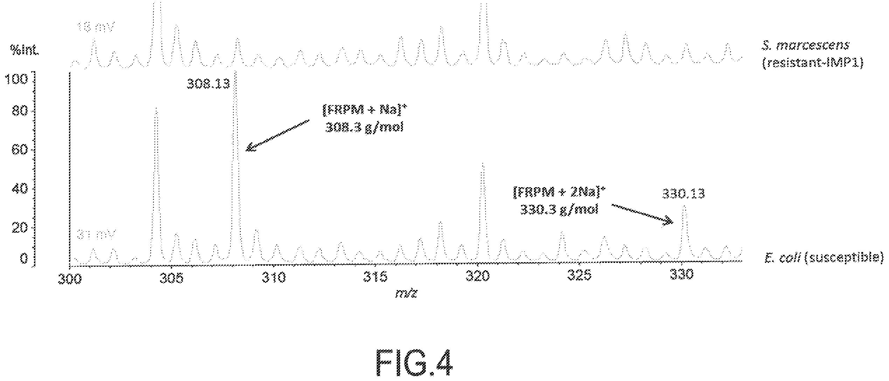

La **Figure 4** présente les spectres de masse obtenus lors de la deuxième série d'acquisitions et montre que l'activité carbapénémase a pu être détectée par MALDI-TOF dans ces conditions grâce à la deuxième série d'acquisitions. Bien que les formes hydrolysées du faropénème ne soient pas détectées dans cette expérience, il a été possible de mettre en évidence la perte des deux formes natives du faropénème, due à la production par les bactéries de carbapénémase.

Après l'acquisition dans les faibles masses, avec la souche sensible un pic majoritaire à 308,13 m/z et un pic minoritaire à 330,13 m/z ont été observés, correspondant respectivement aux deux formes natives du faropénème (correspondant à des masses calculées de 308,3 et 330,3 m/z). Ces deux pics ont été perdus dans le cas où le faropénème avait été incubé avec la souche S. *marcescens* produisant la carbapénémase IMP-1.

Après la première acquisition et l'obtention des spectres, les deux souches ont pu être correctement identifiées avec un haut niveau de confiance. De manière similaire à l'exemple 2, la présence de faropénème dans les zones de caractérisation n'a pas affectée la capacité d'identification de l'espèce par MALDI-TOF et la possibilité de discriminer les bactéries de type E. *coli* des bactéries de type *marcescens,* comme le montrent les résultats obtenus avec la première série d'acquisition, présentés dans le **Tableau 4.**

**Tableau 4**

| **Souche de bactéries/ N° de référence** | **Nombre de pics utilisés par l'identification** | **Espèces** | **Probabilité après comparaison des spectres de référence** |
|---|---|---|---|
| 5 | 100 | *S. marcescens* | 99,9 |
| 5 | 113 | *S. marcescens* | 99,9 |
| 6 | 1 09 | *E. coli* | 99,9 |
| 6 | 101 | *E. coli* | 99,9 |

### Exemple 4.

Cet essai a été réalisé pour augmenter l'adhérence de l'antibiotique sur la zone d'analyse de la plaque MALDI. En effet, le séchage d'une solution d'antibiotique déposée sur la plaque conduit à la formation d'un film qui adhère faiblement à la surface. L'heptakis(2,6-di-*O*-méthyl)-β-cyclodextrine (Heptakis, Sigma-Aldrich ref H0513) a été utilisée pour coller le faropénème à la surface de la plaque MALDI. Au-delà de ses propriétés d'adhérence, le choix de l'Heptakis a été motivé par son poids moléculaire de 1331,36 g.mol⁻¹ qui n'interfère pas avec les pics de masse du faropénème ou de ses produits hydrolysés. Au cours de cette expérience, deux ratios massiques [Heptakis]/[faropénème] ont été testés pour évaluer l'adhérence du faropénème séché et l'impact de l'Heptakis sur la détection des pics natifs et hydrolysés du faropénème.

Les souches de bactéries utilisées sont détaillées dans le **Tableau 5** ci-dessous.

**Tableau 5**

| **Souche de bactéries/ N° de référence** | **Espèces** | **Phénotype** | **Facteur de virulence** |
|---|---|---|---|
| 7 | *K. pneumoniae* | résistant | KPC carbapénémase |
| 8 | *E. coli* ATCC 25922 | sensible | pas de β-lactamase (sauvage) |

Les essais ont été réalisés en mettant en oeuvre les étapes ci-dessous :
- 2 solutions contenant un mélange d'Heptakis et de faropénème et 1 solution de faropénème sans Heptakis ont été prépareés dans un tampon NaCl (0,45%) supplémenté en zinc (sulfate de zinc 0,76 mM). Les concentrations finales en Heptakis et faropénème de ces solutions sont les suivantes :
   ∘ 0 mg/ml d'Heptakis et 1 mg/ml de faropénème,
   ∘ 0,1 mg/ml d'Heptakis et 1 mg/ml de faropénème (ratio massique 1:10),
   ∘ 0,2 mg/ml d'Heptakis et 1 mg/ml de faropénème (ratio massique 1:5),
- 2 µl de chaque solution ont été déposés sur les zones d'analyse de la plaque MALDI (conforme à celle de l'exemple 1),
- la plaque a été incubée à température ambiante, jusqu'à ce que les gouttes de la solution déposée soient complètement sèches,
- pour évaluer l'adhérence, chaque zone d'analyse fonctionnalisée avec le faropénème ou le mélange Heptakis/faropénème a été grattée à l'aide d'une oese pour mimer le dépôt d'une colonie et une photo de la lame a été prise,
- pour évaluer l'effet de la concentration en Heptakis sur la détection des pics du faropénème, une portion de colonie obtenue suite à une croissance pendant 24h sur un milieu gélosé a été déposée en quadriplicat sur des zones d'analyse porteuses de faropénème seul ou de mélanges de faropénème et d'Heptakis,
- la plaque a été incubée à 37°C pendant 2h dans une atmosphère humide,
- 1 µl d'une matrice d'HCCA a été ajouté sur les zones d'analyse porteuses, à la fois, de l'antibiotique ou des mélanges Heptakis/antibiotique et des bactéries,
- une analyse par spectrométrie de masse MALDI-TOF a été réalisée avec un appareillage VITEK^{®} MS, pour une acquisition de spectres dans les faibles masses,
- les pics de la forme native à 308,3 m/z (faropénème + Na) et hydrolysée à 304.3 m/z (faropénème hydrolysé + H) du faropénème, ainsi que le pic de l'HCCA à 212.03 m/z ont été visualisés,
- pour toutes les conditions testées, les intensités des 3 pics ont été enregistrées et les ratios 308/212 et 304/212 ont été calculés. Le pic de l'HCCA à 212 m/z a ici été utilisé comme un pic contrôle d'intensité invariable.

Tous les échantillons ont été analysés en quadriplicat sur la plaque.

**La** **Figure 5** montre l'aspect des zones d'analyse (spots) avant et après grattage avec une oese. La photo met en évidence un bon étalement, après grattage, du mélange Heptakis/faropénème sur toute la surface du spot pour des ratios [Heptakis]/[faropénème] de 1/10 et 1/5, avec une bonne stabilité après grattage. L'antibiotique séché sans Heptakis n'est pas très stable sur la surface de la lame et le film est totalement ou partiellement décroché lors du grattage par l'oese.

La **Figure 6** montre la variation des ratios d'intensités des pics du faropénème natif et du faropénème hydrolysé, par rapport au pic contrôle de l'HCCA en fonction des ratios [Heptakis]/[faropénème] utilisés lors du séchage de l'antibiotique sur la lame, Les valeurs représentent la moyenne de quatre spots. Pour des ratios de 1/10 et 1/5, la détection est comparable à la condition sans Heptakis avec un avantage supplémentaire de reproductibilité en présence d'Heptakis. En effet, la forte variabilité observée en absence d'Heptakis (barre d'erreur importante) est due à la perte totale ou partielle de l'antibiotique sur certains spots lors du dépôt de la colonie. En ce qui concerne l'apparition du pic hydrolysé suite à une incubation avec la souche productrice de carbapénémase de type KPC, nous observons le même phénomène c'est-à-dire une bonne détection avec des ratios [Heptakis]/[faropénème] de 1/10 et 1/5.

Les résultats de cette expérience montrent que l'utilisation de l'Heptakis à des concentrations adaptées (0,1 ou 0,2 mg/ml pour 1 mg/ml de faropénème) permet de stabiliser l'antibiotique sur la lame pour le stockage tout en assurant un mélange optimal avec la bactérie lors du dépôt de la colonie. A ces mêmes concentrations, l'Heptakis permet également d'améliorer la reproductibilité des résultats entre les spots et n'interfère pas avec la détection des pics ou encore avec la réaction d'hydrolyse du faropénème,

### Exemple 5.

Cet essai a été mené pour évaluer la possibilité de réaliser la réaction d'hydrolyse sur la zone d'analyse fonctionnalisée de la plaque MALDI à partir d'un dépôt liquide c'est-à-dire un inoculum bactérien. En effet, la difficulté rencontrée lors du dépôt en colonie est le manque de normalisation par rapport à la quantité de microorganismes déposés et la variabilité du dépôt selon le manipulateur. Ainsi, le dépôt d'une colonie sur lame pour une application d'identification classique en MALDI-TOF nécessite une formation technique qui n'élimine pas complètement le risque de variabilité des résultats dû à des dépôts hétérogènes. Aussi, un inoculum bactérien de concentration connue a été appliqué sur les zones d'analyse fonctionnalisées de la plaque MALDI.

Les souches de bactéries utilisées sont détaillées dans le **Tableau 5** ci-dessus.

Les essais ont été réalisés en mettant en oeuvre les étapes ci-dessous :
- 2 µl d'une solution de faropénème à 1 mg/ml préparé dans un tampon NaCl (0,45%) supplémenté en zinc (sulfate de zinc 0,76 mM) ont été déposés sur les zones d'analyse de la plaque MALDI et séchés à température ambiante,
- 2 µl d'inocula bactériens à des concentrations de 3 ou 6 McFarland ont été appliqués en quadriplicat sur les zones d'analyses fonctionnalisées,
- la plaque a été incubée à 37°C pendant 2h dans une atmosphère humide,
- 1 µl d'une matrice d'HCCA a été ajouté sur les zones d'analyse porteuses, à la fois, de l'antibiotique et des bactéries,
- une analyse par spectrométrie de masse MALDI-TOF a été réalisée avec un appareillage VITEK^{®} MS, pour une double acquisition de spectres dans les faibles masses pour observer les pics du faropénème et dans les hautes masses pour l'identification (conforme à l'exemple 2),
- les pics des formes natives à 308.3 m/z (faropénème + Na) et à 330.3 m/z (faropénème + 2Na) ainsi que le pic de la forme hydrolysée à 304.3 m/z (faropénème hydrolysé + H) ont été visualisés,
- pour toutes les conditions testées, les intensités des 3 pics ont été enregistrées et les ratios 304/308 et 304/330 ont été calculés pour quantifier l'hydrolyse du faropénème.

Tous les échantillons ont été analysés en quadriplicat sur la plaque.

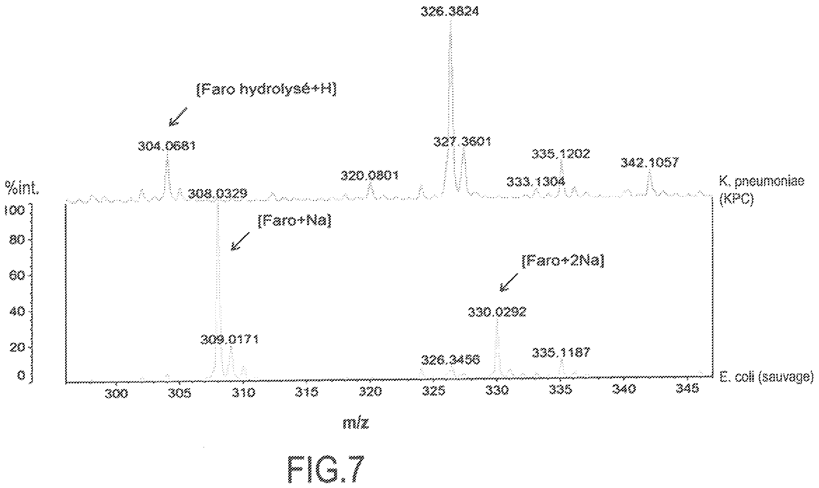

La **Figure 7** présente les spectres de masse obtenus lors de la deuxième série d'acquisitions et montre que l'activité carbapénémase a pu être détectée par MALDI-TOF suite à un dépôt d'inocula bactériens à 6 McFarland. Il a en effet été possible de mettre en évidence la perte des deux formes natives et l'apparition de la forme hydrolysée du faropénème, due à la production par les bactéries de carbapénémase.

Après l'acquisition dans les faibles masses, avec la souche sensible un pic majoritaire à 308,03 m/z et un pic minoritaire à 330,02 m/z ont été observés, correspondant respectivement aux deux formes natives du faropénème (correspondant à des masses calculées de 308,3 et 330,3 m/z). Ces deux pics ont été perdus dans le cas où le faropénème avait été incubé avec la souche *K. pneumoniae* produisant la carbapénémase KPC et on voit l'apparition d'un pic à 304.06 m/z correspondant à la forme hydrolysée du faropénème (masse calculée de 304,03).

Après la première acquisition et l'obtention des spectres, les deux souches ont pu être correctement identifiées avec un haut niveau de confiance. La présence de faropénème dans les zones de caractérisation et le dépôt en inoculum n'ont pas affecté la capacité d'identification de l'espèce par MALDI-TOF et la possibilité de discriminer les bactéries de type *E. coli* des bactéries de type *K. pneumoniae,* comme le montrent les résultats obtenus avec la première série d'acquisitions, présentés dans le **Tableau 6.**

**Tableau 6**

| **Souche de bactéries/ N° de référence** | **Nombre de pics utilisés par l'identification** | **Espèces** | **Probabilité après comparaison des spectres de référence** |
|---|---|---|---|
| 7 | 177 | *K. pneumoniae* | 91,2 |
| 8 | 149 | *E. coli* | 93 |

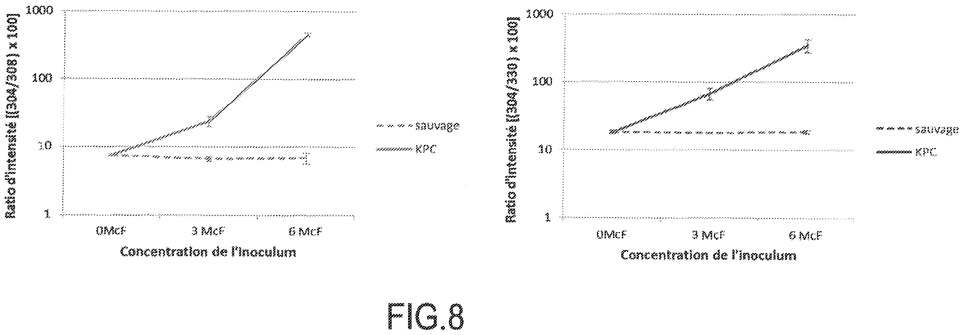

La **Figure 8** montre la variation des ratios d'intensités 304/308 et 304/330 en fonction de la concentration en inoculum utilisée pour les 2 souches testées. Pour la souche sauvage qui n'hydrolyse pas l'antibiotique, aucune variation significative des 2 ratios n'a été observée quelle que soit la concentration de l'inoculum bactérien. Pour la souche productrice de carbapénémase de type KPC, une augmentation significative des 2 ratios, qui est proportionnelle à la concentration de l'inoculum, est observée. Cette augmentation des ratios se traduit par une diminution d'intensité des pics natifs et une augmentation d'intensité du pic hydrolysé tel que représenté sur la **Figure 7****.**

Les résultats de cette expérience montrent que le dépôt en inoculum est également adapté à la réaction d'hydrolyse sur plaque MALDI et à l'identification par spectrométrie de masse MALDI-TOF. De plus, les faibles barres d'erreurs observées sur une moyenne de 4 dépôts suggèrent une très bonne reproductibilité des résultats,

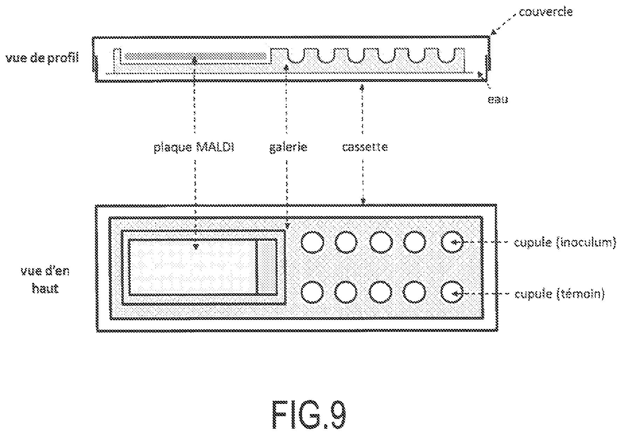

La **Figure 9** représente un modèle de boitier intégrant une plaque MALDI qui pourrait être adapté au dépôt d'une population de micro-organisme sous forme liquide. Une lame plaque MALDI dont les zones d'analyse sont déjà recouvertes d'antibiotique séché est placée dans une galerie à l'intérieur de laquelle ont été moulées plusieurs cupules (au nombre de 12 dans le modèle présenté). Les 6 cupules alignées sur un même axe contiennent un témoin d'opacité sous une forme déshydratée et les 6 autres cupules sont vides. La galerie contenant la lame est elle-même placée dans une cassette possédant un couvercle. Pour des besoins de stockage, cette cassette peut notamment être emballée de manière hermétique, à l'abri de la lumière et de l'humidité.

Le protocole d'utilisation de ce produit peut être décrit par les étapes suivantes :
- enlever le couvercle de la cassette,
- remplir les 12 cupules avec un volume de 50 à 100 µl d'eau ou de tampon physiologique. Le remplissage des 6 cupules contenants le témoin d'opacité entraîne la formation de solutions turbides,
- préparer des inocula dans les 6 autres cupules en ajoutant une colonie ou une portion de colonie de façon à obtenir une turbidité comparable au témoin d'opacité de la cupule d'en face,
- déposer 2 µl de chaque inoculum sur les zones d'analyses porteuses de l'antibiotique,
- ajouter de l'eau à l'aide d'une pipette dans la cassette pour créer une atmosphère humide,
- remettre le couvercle et incuber à 37°C pendant 1 à 2h (ou moins),
- ajouter 1 µl d'une matrice d'HCCA sur les zones d'analyse porteuses, à la fois, de l'antibiotique et des bactéries,
- récupérer la lame pour une analyse par spectrométrie de masse MALDI-TOF.

L'ajout du témoin d'opacité permet d'éviter la préparation d'inoculum en utilisant un appareil de mesure de densité ou de turbidité et permet donc un gain de temps. Par ailleurs, les appareils de mesure couramment utilisés en routine, tels que les densitomètres, sont adaptés à des volumes plus importants (de 1 à 3 ml) nécessitant donc l'utilisation d'une quantité plus importante de bactéries.

La préparation de l'inoculum pourrait également se faire en utilisant un extrait solide ou liquide de bactéries obtenues directement à partir d'un échantillon biologique (ex : bactéries extraites d'une hémoculture).

Le modèle présenté sur **la** **Figure 9****,** permet de tester 6 souches différentes. Ce modèle peut être adapté en fonction la quantité de souches testées en routine notamment en augmentant le nombre de cupules.

### Références :

Hooff, G, P., J, J. van Kampen, R. J. Meesters, A. van Belkum, W. H. Goessens and T. M. Luider (2012). "Characterization of beta-lactamase enzyme activity in bacterial lysates using MALDI-mass spectrometry." J Proteome Res 11(1): 79-84.
Hrabak, J., V. Studentova, R, Walkova, H. Zemlickova, V. Jakubu, E. Chudackova, M. Gniadkowski, Y. Pfeifer, J. D. Perry, K. Wilkinson and T. Bergerova (2012). "Detection of NDM-1, VIM-1, KPC, OXA-48, and OXA-162 carbapenemases by matrix-assisted laser desorption ionization-time of flight mass spectrometry." J Clin Microbiol 50(7): 2441-2443.
Hrabak, J., R. Walkova, V. Studentova, E. Chudackova and T. Bergerova (2011). "Carbapenemase activity detection by matrix-assisted laser desorption ionization-time of flight mass spectrometry." J Clin Microbiol 49(9): 3222-3227.
Sparbier, K., S. Schubert, U, Weller, C. Boogen and M. Kostrzewa (2012). "Matrix-assisted laser desorption ionisation-time of flight mass spectrometry-based functional assay for rapid détection of résistance against beta-lactam antibiotics." J Clin Microbiol 50(3): 927-937.

## Revendications

1. Procédé de caractérisation d'une population d'au moins un micro-organisme, qui comprend, en amont de la caractérisation, la préparation d'une zone de caractérisation d'une plaque d'analyse pour la réalisation de l'au moins une caractérisation selon les étapes successives suivantes :
- une étape consistant à disposer d'une plaque d'analyse pour une caractérisation par la technique MALDI, comprenant au moins une zone d'analyse porteuse d'un agent antimicrobien,
- une étape de dépose de la population d'au moins un micro-organisme sur ladite zone d'analyse au contact de l'agent antimicrobien,
- une étape d'incubation, consistant à conserver la plaque d'analyse, dans des conditions et pendant un temps suffisant, pour permettre l'interaction de l'agent antimicrobien et du micro-organisme présent,
- une étape de dépose sur la zone d'analyse, d'une matrice adaptée à la technique MALDI,
dans lequel, pour la caractérisation, la population d'au moins un micro-organisme déposée sur la zone de caractérisation est, ensuite, soumise à au moins une étape d'ionisation par bombardement de la zone de caractérisation par un faisceau laser selon la technique de spectrométrie de masse par désorption-ionisation assistée par matrice nommée MALDI, ladite caractérisation comprenant :
- l'identification de la famille, du genre, ou, de préférence, de l'espèce d'une population d'un micro-organisme déposée sur la zone de caractérisation, qui met en oeuvre une première analyse par spectrométrie de masse de type MALDI correspondant à une première étape d'ionisation de la zone de caractérisation, et utilisant pour l'analyse une première calibration, et
- la détermination de la présence éventuelle sur la zone de caractérisation d'une population d'un micro-organisme résistant à l'agent antimicrobien qui met en oeuvre une deuxième analyse par spectrométrie de masse selon la technique MALDI-TOF correspondant à une seconde étape d'ionisation de la même zone de caractérisation, et utilisant pour l'analyse une deuxième calibration.

2. Procédé de caractérisation selon la revendication 1, **caractérisé en ce qu'**une population d'un seul micro-organisme à caractériser est déposée.

3. Procédé de caractérisation selon la revendication 1 ou 2, **caractérisé en ce que** la population de micro-organisme(s) déposée est préparée sans étape de mise en contact préalable avec un agent antimicrobien.

4. Procédé de caractérisation selon l'une quelconque des revendications 1 à 3, **caractérisé en ce que** la population de micro-organisme(s) est obtenue après une étape de concentration, enrichissement et/ou purification et/ou correspond à une colonie ou à une fraction de colonie obtenue après croissance sur un milieu adapté, notamment un milieu gélosé.

5. Procédé de caractérisation selon l'une quelconque des revendications 1 à 4, **caractérisé en ce que** l'agent antimicrobien est sélectionné de manière à permettre l'identification de la résistance due à la production de bêta-lactamase, et notamment de carbapénémase.

6. Procédé de caractérisation selon l'une quelconque des revendications 1 à 5, **caractérisé en ce que** l'agent antimicrobien est un antibiotique, de préférence sélectionné parmi les pénicillines, les céphalosporines, les céphamycines, les carbapénèmes, les monobactames et en particulier parmi l'ampicilline, l'amoxicilline, la ticarcilline, la piperacilline, la céfalotine, le céfuroxime, la céfoxitine, le céfixime, le céfotaxime, la céftazidime, la ceftriaxone, la cefpodoxime, le céfépime, l'aztreonam, l'ertapénème, l'imipénème, le méropénème, le faropénème.

7. Procédé de caractérisation selon l'une quelconque des revendications 1 à 6, **caractérisé en ce qu'**il comprend une étape de fonctionnalisation conduisant à l'obtention de la plaque d'analyse pour une caractérisation par la technique MALDI, comprenant au moins une zone d'analyse porteuse d'un agent antimicrobien, ladite étape de fonctionnalisation étant, de préférence, réalisée par dépôt d'une solution aqueuse de l'agent antimicrobien, suivi d'un séchage.

8. Procédé de caractérisation selon l'une quelconque des revendications 1 à 7, caractérisé en ce la ou les zone(s) d'analyse fonctionnalisée(s), est(sont) porteuse(s) d'un seul agent antimicrobien.

9. Procédé de caractérisation selon la revendication 1, **caractérisé en ce que** l'analyse par spectrométrie de masse selon la technique MALDI-TOF, permettant de conclure si une population d'un micro-organisme résistant à l'agent antimicrobien est présente sur la zone de caractérisation, consiste à vérifier la présence de l'agent antimicrobien et/ou d'un produit de dégradation de l'agent antimicrobien.

10. Procédé de caractérisation selon l'une des revendications 1 à 9 **caractérisé en ce que** l'identification de la famille, du genre, ou, de préférence, de l'espèce d'une population d'un micro-organisme, et la détermination de la résistance éventuelle à l'agent antimicrobien concerne le même micro-organisme.

## Patentansprüche

1. Verfahren zur Charakterisierung einer Population mindestens eines Mikroorganismus, das vor der Charakterisierung die Vorbereitung eines Charakterisierungsbereichs einer Analyseplatte zur Durchführung der mindestens eine Charakterisierung gemäß den folgenden aufeinanderfolgenden Schritten aufweist:
- einem Schritt, der darin besteht, eine Analyseplatte für eine Charakterisierung durch die MALDI-Technik anzuordnen, die mindestens einen Analysebereich, der ein antimikrobielles Mittel trägt, aufweist,
- einem Schritt des Abscheidens der Population von mindestens einem Mikroorganismus auf dem Analysebereich in Kontakt mit dem antimikrobiellen Mittel,
- einem Schritt der Inkubation, der darin besteht, die Analyseplatte unter Bedingungen und für eine ausreichende Zeit aufzubewahren, um die Wechselwirkung des antimikrobiellen Mittels und des vorhandenen Mikroorganismus zu ermöglichen,
- einem Schritt des Abscheidens einer Matrix auf dem Analysebereich, die für die MALDI-Technik geeignet ist,
wobei für die Charakterisierung die Population von mindestens einem Mikroorganismus, der auf dem Charakterisierungsbereich abgeschieden wird, dann mindestens einem lonisierungsschritt durch Beschuss des Charakterisierungsbereichs mit einem Laserstrahl gemäß der Massenspektrometrietechnik durch Matrix assistierte Laser-Desorption/Ionisation, die MALDI genannt wird, unterzogen wird, wobei die Charakterisierung aufweist:
- das Identifizieren der Familie, der Gattung oder vorzugsweise der Spezies einer Population eines Mikroorganismus, die auf dem Charakterisierungsbereich abgeschieden wird, das eine erste Analyse durch Massenspektrometrie vom Typ MALDI durchführt, die einem ersten lonisierungsschritt des Charakterisierungsbereichs entspricht und für die Analyse eine erste Kalibrierung verwendet, und
- das Bestimmen der eventuellen Anwesenheit in dem Charakterisierungsbereich einer Population eines Mikroorganismus, die gegen das antimikrobielle Mittel resistent ist, das eine zweite Analyse durch Massenspektrometrie gemäß der MALDI-TOF-Technik durchführt, die einem zweiten lonisierungsschritt des gleichen Charakterisierungsbereichs entspricht und für die Analyse eine zweite Kalibrierung verwendet.

2. Verfahren zur Charakterisierung nach Anspruch 1, **dadurch gekennzeichnet, dass** eine Population eines einzigen zu charakterisierenden Mikroorganismus abgeschieden wird.

3. Verfahren zur Charakterisierung nach Anspruch 1 oder 2, **dadurch gekennzeichnet, dass** die abgeschiedene Population des (der) Mikroorganismus (Mikroorganismen) ohne den Schritt des vorherigen Inkontaktbringens mit einem antimikrobiellen Mittel hergestellt wird.

4. Verfahren zur Charakterisierung nach einem der Ansprüche 1 bis 3, **dadurch gekennzeichnet, dass** die Population des (der) Mikroorganismus (Mikroorganismen) nach einem Schritt des Konzentrierens, Anreicherns und/oder Reinigens erhalten wird und/oder einer Kolonie oder einem Bruchteil einer Kolonie entspricht, die nach dem Wachstum auf einem geeigneten Medium, insbesondere einem Agarmedium, entspricht.

5. Verfahren zur Charakterisierung nach einem der Ansprüche 1 bis 4, **dadurch gekennzeichnet, dass** das antimikrobielle Mittel derart ausgewählt wird, um das Identifizieren der Resistenz auf Grund der Produktion von Beta-Lactamase, und insbesondere von Carbapenemase, ermöglicht.

6. Verfahren zur Charakterisierung nach einem der Ansprüche 1 bis 5, **dadurch gekennzeichnet, dass** das antimikrobielle Mittel ein Antibiotikum ist, vorzugsweise ausgewählt unter Penicillinen, Cephalosporinen, Cephamycinen, Carbapenemen, Monobactamen und insbesondere unter Ampicillin, Amoxicillin, Ticarcillin, Piperacillin, Cephalothin, Cefuroxim, Cefoxitin, Cefixim, Cefotaxim, Ceftazidim, Ceftriaxon, Cefpodoxim, Cefepim, Aztreonam, Ertapenem, Imipenem, Meropenem, Faropenem.

7. Verfahren zur Charakterisierung nach einem der Ansprüche 1 bis 6, **dadurch gekennzeichnet, dass** es einen Funktionalisierungsschritt aufweist, der zum Erhalten der Analyseplatte für eine Charakterisierung durch die MALDI-Technik führt, die mindestens einen Analysebereich, der ein antimikrobielles Mittel trägt, aufweist, wobei der Funktionalisierungsschritt vorzugsweise durch Abscheiden von einer wässrigen Lösung des antimikrobiellen Mittels, gefolgt von einem Trocknen durchgeführt wird.

8. Verfahren zur Charakterisierung nach einem der Ansprüche 1 bis 7, **dadurch gekennzeichnet, dass** der (die) funktionalisierte (n) Analysebereich (e) ein einziges antimikrobielles Mittel trägt (tragen).

9. Verfahren zur Charakterisierung nach einem der Ansprüche 1 bis 8, **dadurch gekennzeichnet, dass** die Analyse durch Massenspektrometrie gemäß der MALDI-TOF-Technik, die ermöglicht, zu beurteilen, ob eine Population eines Mikroorganismus, die gegen das antimikrobielle Mittel resistent ist, auf dem Charakterisierungsbereich vorhanden ist, darin besteht, die Anwesenheit des antimikrobiellen Mittels und/oder eines Abbauproduktes des antimikrobiellen Mittels zu überprüfen.

10. Verfahren zur Charakterisierung nach einem der Ansprüche 1 bis 9, **dadurch gekennzeichnet, dass** das Identifizieren der Familie, der Gattung oder vorzugsweise der Spezies einer Population eines Mikroorganismus und das Bestimmen der eventuellen Resistenz gegen das antimikrobielle Mittel den gleichen Mikroorganismus betrifft.

## Claims

1. A method of characterizing a population of at least one microorganism which comprises, before the caracterization, the preparation of a characterization zone of an analysis plate in order to carry out the at least one characterization, the said preparation comprising the following steps in succession:
- a step consisting in providing an analysis plate for a characterization by means of the MALDI technique, the plate comprising at least one analysis zone carrying an antimicrobial agent;
- a step of depositing the population of at least one microorganism onto said analysis zone in contact with the antimicrobial agent;
- an incubation step, consisting in preserving the analysis plate under conditions and for a sufficient time to allow the antimicrobial agent and the microorganism that is present to interact; and
- a step of depositing a matrix that is suitable for the MALDI technique onto the analysis zone,
in which, the population of at least one microorganism deposited on the analysis zone, then, undergoes at least one ionization step by bombarding the analysis zone with a laser beam in accordance with the matrix-assisted laser desorption-ionization mass spectrometry technique known as MALDI,
the said caracterizing comprising:
• identifying the family, genus, or, as is preferable, the species of a population of a microorganism deposited on the characterization zone by carrying out a first analysis by MALDI type mass spectrometry corresponding to a first step of ionizing the characterization zone, and by using a first calibration for the analysis; and
• determining the possible presence on the characterization zone of a population of a microorganism that is resistant to the antimicrobial agent by carrying out a second analysis by mass spectrometry using the MALDI-TOF technique corresponding to a second step of ionizing the same characterization zone, and by using a second calibration for the analysis.

2. The characterization method according to claim 1, **characterized in that** a population of a single microorganism to be characterized is deposited.

3. The characterization method according to claim 1 or claim 2, **characterized in that** the deposited population of microorganism(s) is prepared without a prior step of contact with an antimicrobial agent.

4. The characterization method according to any one of claims 1 to 3, **characterized in that** the population of microorganism(s) is obtained after a step of concentration, enrichment and/or purification and/or corresponding to a colony or to a fraction of a colony obtained after growth on a suitable medium, in particular a gel medium.

5. The characterization method according to any one of claims 1 to 4, **characterized in that** the antimicrobial agent is selected in a manner such as to allow the resistance due to the production of beta-lactamase, and in particular carbapenemase, to be identified.

6. The characterization method according to any one of claims 1 to 5, **characterized in that** the antimicrobial agent is an antibiotic, preferably selected from penicillins, cephalosporins, cephamycins, carbapenems, monobactams and in particular from ampicillin, amoxicillin, ticarcillin, piperacillin, cefalotin, cefuroxime, cefoxitin, cefixime, cefotaxime, ceftazidime, ceftriaxone, cefpodoxime, cefepime, aztreonam, ertapenem, imipenem, meropenem, and faropenem.

7. The characterization method according to any one of claims 1 to 6, **characterized in that** it comprises a functionalization step in order to obtain the analysis plate for a characterization by means of the MALDI technique comprising at least one analysis zone carrying an antimicrobial agent, said functionalization step preferably having been carried out by depositing an aqueous solution of the antimicrobial agent, followed by drying.

8. The characterization method according to any one of claims 1 to 7, **characterized in that** the functionalized analysis zone(s) carry(ies) a single antimicrobial agent.

9. The characterization method according to claim 1, **characterized in that** the analysis by mass spectrometry using the MALDI-TOF technique in order to conclude whether a population of a microorganism that is resistant to the antimicrobial agent is present on the characterization zone consists in verifying the presence of the antimicrobial agent and/or of a degradation product of the antimicrobial agent.

10. The characterization method according to any one of claims 1 to 9, **characterized in that** identifying the family, genus, or, as is preferable, the species of a population of a microorganism and determining whether the possible presence of the antimicrobial agent concerns the same microorganism.
